(19) **Europäisches Patentamt / European Patent Office / Office européen des brevets**

(11) **EP 4 406 503 B1**

(12) ## EUROPEAN PATENT SPECIFICATION

(45) Date of publication and mention
of the grant of the patent:
**08.07.2026 Bulletin 2026/28**

(21) Application number: **23315014.3**

(22) Date of filing: **27.01.2023**

(51) International Patent Classification (IPC):
**A61B 34/20** (2016.01)    **A61B 34/30** (2016.01)

(52) Cooperative Patent Classification (CPC):
**A61B 34/20; A61B 34/30;** A61B 2034/2055;
A61B 2090/0818

(54) **SURGICAL NAVIGATION SYSTEM DEALING WITH PARTIALLY-OBSCURED MARKERS**

CHIRURGISCHES NAVIGATIONSSYSTEM MIT HANDHABUNG VON TEILWEISE VERBORGENEN MARKERN

SYSTÈME DE NAVIGATION CHIRURGICALE TRAITANT DES MARQUEURS PARTIELLEMENT DISSIMULÉS

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(43) Date of publication of application:
**31.07.2024 Bulletin 2024/31**

(73) Proprietor: **Ecential Robotics**
**38610 Gieres (FR)**

(72) Inventor: **Sieffert, Jérôme**
**38610 GIERES (FR)**

(74) Representative: **INNOV-GROUP**
**209 Avenue Berthelot**
**69007 Lyon (FR)**

(56) References cited:
US-A- 5 828 770      US-A1- 2016 242 858
US-A1- 2021 401 510      US-A1- 2022 409 288

## Description

## Technical field

The invention relates to the technical field of surgical navigation systems.

The invention is notably applicable to navigate a surgical tool during a surgical intervention.

## Background art

A surgical navigation system known from prior art, comprises:

- a surgical tool;
- an optical tracker, arranged to track a pose of the surgical tool, and having fiducial markers;
- a stereoscopic camera, arranged to capture images of the fiducial markers;
- a data processor, configured to identify the fiducial markers and determine the positions of the fiducial markers, from the images captured by the stereoscopic camera.

In case at least one of the fiducial markers is partially obscured (e.g. physical interposition, defective coating etc.) the two optical sensors of the stereoscopic camera will give divergent information about the position of the corresponding fiducial marker. The data processor is configured to apply a criterion based on a root-mean-square (RMS) deviation to compare the information of the two optical sensors of the stereoscopic camera, so as to complete an image registration of the images captured by the stereoscopic camera. The image registration is completed as long as the image registration error (i.e. the RMS error) is below a threshold.

Such a surgical navigation system from the prior art is not entirely satisfactory because the completion of the image registration using the RMS error on positions of the fiducial marker does not guarantee clinical relevance. Indeed, it has been found that an offset of the tip of a surgical instrument of several centimeters may not be detected by using the RMS error. This is due to the fact that the non-obscured fiducial markers may compensate in the calculation of the RMS deviation of the positions of the partially-obscured fiducial marker.

The skilled person in the art is looking for a more reliable criterion than the RMS error on the positions of the partially-obscured fiducial marker, and having a relevant clinical meaning in order to improve the safety of the navigation system.

US 2016/242858 A1 discloses a navigation system and a method for reducing tracking interruptions during a surgical procedure. US 2022/409288 A1 discloses an optical navigation system for determining the position of an anatomy of interest in order to optimally position a medical instrument. US 2021/401510 A1 discloses surgical navigation procedures directed to dynamic reference arrays, which include radio-opaque fiducial markers and tracking markers.

## Disclosure of the invention

The invention aims to completely or partly overcome the aforementioned drawbacks. To this end, one subject of the invention is a surgical navigation system, comprising:

- a surgical tool, having a reference zone;
- an optical tracker, arranged to track a pose of the surgical tool, and having fiducial markers;
- a stereoscopic camera, arranged to capture images of $N_{tot}$ fiducial markers, where $N_{tot}$ is an integer superior or equal to 4;
- a data processor, configured to perform steps:

  a) identify the $N_{tot}$ fiducial markers and determine the positions of the $N_{tot}$ fiducial markers, from the images captured by the stereoscopic camera;
  b) generate a set of patterns, each pattern having M fiducial markers, where M is an integer superior or equal to 3 and strictly inferior to $N_{tot}$; the set of patterns including N fiducial markers, where N is an integer inferior or equal to $N_{tot}$;
  c) for each pattern of the set generated in step b):

  - extract the positions of the corresponding M fiducial markers thereof from step a);
  - compute a pose of the reference zone, in a reference coordinate system, from the extracted positions of the corresponding M fiducial markers;

  d) apply a criterion to compare the poses of the reference zone computed in step c) therebetween for the set of patterns, the criterion being designed to detect a presence of at least one partially-obscured fiducial marker within the N fiducial markers.

Thus, such a surgical navigation system according to the invention makes it possible to reliably detect the presence of at least one partially-obscured fiducial marker by comparing poses of the reference zone (e.g. a tip of a surgical instrument), which have clinical significance. More specifically, the criterion applied in step d) compares the poses of the reference zone computed in step c) therebetween for the set of patterns.

Another subject of the invention is a surgical navigation system, comprising:

- a surgical tool, having a reference zone;
- an optical tracker, arranged to track a pose of the surgical tool, and having fiducial markers;
- a stereoscopic camera, arranged to capture images

of $N_{tot}$ fiducial markers, where $N_{tot}$ is an integer superior or equal to 4;
- a data processor, configured to perform steps:

> a) identify the $N_{tot}$ fiducial markers and determine the positions of the $N_{tot}$ fiducial markers, from the images captured by the stereoscopic camera;
> b) generate a set of patterns, each pattern having M fiducial markers, where M is an integer superior or equal to 3 and strictly inferior to $N_{tot}$; the set of patterns including N fiducial markers, where N is an integer inferior or equal to $N_{tot}$;
> c) for each pattern of the set generated in step b):
>
> > - extract the positions of the corresponding M fiducial markers thereof from step a);
> > - compute a pose of the reference zone, in a reference coordinate system, from the extracted positions of the corresponding M fiducial markers;
>
> c') extract the positions of the N fiducial markers from step a); and compute a pose of the reference zone, in the reference coordinate system, from the extracted positions of the N fiducial markers;
> d) apply a criterion to compare the pose of the reference zone computed in step c'), with the poses of the reference zone computed in step c) for the set of patterns, the criterion being designed to detect a presence of at least one partially-obscured fiducial marker within the N fiducial markers.

Thus, such a surgical navigation system according to the invention makes it possible to reliably detect the presence of at least one partially-obscured fiducial marker by comparing poses of the reference zone (e.g. a tip of a surgical instrument), which have clinical significance. More specifically, the criterion applied in step d) compares the pose of the reference zone computed in step c'), with the poses of the reference zone computed in step c) for the set of patterns.

The surgical navigation system according to the invention may include one or more of the following features.

According to one feature of the invention:

- the stereoscopic camera is arranged to capture images of $N_{tot}$ fiducial markers, where $N_{tot}$ is an integer superior or equal to 5;
- each pattern of the set of patterns generated in step b) has M fiducial markers, where M is an integer superior or equal to 4 and strictly inferior to $N_{tot}$;
- the data processor is further configured to perform the following step when the criterion applied in step d) is satisfied:

e) for each pattern of the set generated in step b):

- generate a set of sub-patterns, each sub-pattern having m fiducial markers, where m is an integer equal to M-1;
- compute a pose of the reference zone, in the reference coordinate system, for each sub-pattern of the set, from the positions of the corresponding m fiducial markers thereof extracted in step c);
- apply a criterion to compare the computed poses of the reference zone therebetween for the set of sub-patterns, the criterion being designed to:

> locate $N_{po}$ partially-obscured fiducial markers within the M fiducial markers of the corresponding pattern, if $N_{po} \leq M-3$, where $N_{po}$ is the number of partially-obscured fiducial markers;
> detect a presence of $N_{po}$ partially-obscured fiducial markers within the M fiducial markers of the corresponding pattern, if $N_{po} > M-3$.

Thus, one advantage afforded is that step e) gives more information than a simple presence detection. Indeed, step e) allows to locate $N_{po}$ partially-obscured fiducial markers when $N_{po} \leq M-3$.

Step e) cannot locate $N_{po}$ partially-obscured fiducial markers when $N_{po} > M-3$. However, in this case, step e) gives information on the quantity of partially-obscured fiducial markers. More specifically, step e) cannot give the exact number of partially-obscured fiducial markers, but can give the information that $N_{po} > M-3$.

According to one feature of the invention, the data processor is further configured to perform steps:

> $f_1$) complete an image registration of the images captured by the stereoscopic camera by removing the $N_{po}$ partially-obscured fiducial markers located in step e) when $N_{po} \leq M-3$;
> $f_2$) stop the surgical navigation if the presence of $N_{po}$ partially-obscured fiducial markers is detected in step e) when $N_{po} > M-3$.

Thus, one advantage afforded is that of exploiting the information obtained in step e). More specifically, on the one hand, the location of the $N_{po}$ partially-obscured fiducial markers, when $N_{po} \leq M-3$, makes it possible to reliably complete an image registration of the images captured by the stereoscopic camera. On the other hand, the presence of $N_{po}$ partially-obscured fiducial markers, when $N_{po} > M-3$, switches off the surgical navigation for safety purpose.

According to one feature of the invention, each pattern of the set generated in step b) is selected according to the positions of the corresponding M fiducial markers thereof with respect to each other.

Thus, one advantage afforded is that of improving the reliability of the criterion applied in step d).

According to one feature of the invention, the set of patterns generated in step b) satisfies a spread criterion that compares relative positions of the M fiducial markers for each pattern of the set, from the positions determined in step a); the spread criterion being designed to detect a minimal spatial spread between the M fiducial markers of a given pattern of the set.

Thus, one advantage afforded is that of improving the reliability of the criterion applied in step d).

According to one feature of the invention, each pattern of the set generated in step b) is selected according to the positions of the corresponding M fiducial markers thereof with respect to the stereoscopic camera.

Thus, one advantage afforded is that of improving the reliability of the criterion applied in step d).

According to one feature of the invention, the set of patterns generated in step b) satisfies a visibility criterion that compares visibility levels of the M fiducial markers between all patterns of the set, from the images captured by stereoscopic camera; the visibility criterion being designed to detect a minimal visibility level at which the M fiducial markers of a given pattern of the set are discerned by the stereoscopic camera within a predetermined margin of error.

Thus, one advantage afforded is that of improving the reliability of the criterion applied in step d).

According to one feature of the invention, the set of patterns generated in step b) satisfies both the spread criterion and the visibility criterion.

Thus, one advantage afforded is that of maximizing the reliability of the criterion applied in step d).

According to one feature of the invention, the data processor is configured to perform step b) by setting the integer M of fiducial markers to the integer that is equal to $N_{tot} - 1$.

Thus, one advantage afforded is that of preserving the data processor's computing resources by reducing the number of possible combinations of M fiducial markers among $N_{tot}$ fiducial markers. More specifically, the number of possible combinations, denoted $C_{N_{tot}}^{M}$, is reduced to:

$$C_{N_{tot}}^{M} = C_{N_{tot}}^{N_{tot}-1} = \frac{N_{tot}!}{(N_{tot}-1)!\,1!} = N_{tot}$$

According to one feature of the invention, the data processor is configured to perform step b) by:

$b_1$) setting the integer M of fiducial markers for each pattern of the set;
$b_2$) browsing through all or part of combinations of M fiducial markers from step a), so as to generate patterns.

According to one feature of the invention, the data processor is configured to perform step:
$b_3$) select a set of patterns, from patterns generated in

step $b_2$), that satisfies the spread criterion.

Thus, one advantage afforded is that of improving the reliability of the criterion applied in step d).

According to one feature of the invention, the data processor is configured to perform step:
$b'_3$) select a set of patterns, from patterns generated in step $b_2$), that satisfies the visibility criterion.

Thus, one advantage afforded is that of improving the reliability of the criterion applied in step d).

According to one feature of the invention, the data processor is configured to perform step:
$b''_3$) select a set of patterns, from patterns generated in step $b_2$), that satisfies both the spread criterion and the visibility criterion.

Thus, one advantage afforded is that of maximizing the reliability of the criterion applied in step d).

According to one feature of the invention, the criterion applied in step d) is a spread criterion.

Thus, one advantage afforded is a simplicity of implementation.

According to one feature of the invention, the spread criterion is designed to:

- detect a minimal spatial spread between the poses of the reference zone computed in step c) for the set of patterns, so as to detect the presence of the at least one partially-obscured fiducial marker within the N fiducial markers;
- detect a minimal spatial spread between the pose of the reference zone computed in step c') and each pose of the reference zone computed in step c), so as to detect the presence of the at least one partially-obscured fiducial marker within the N fiducial markers.

### Definitions

- The term "surgical tool" is understood to mean an object used in surgery. The surgical tool may be an instrument or an implant.
- The term "fiducial markers" is understood to mean markers used to locate the position and orientation of the surgical tool in combination with the stereoscopic camera. The fiducial markers are preferably optical markers. The optical markers preferably perform near-infrared optical tracking in combination with the stereoscopic camera. The fiducial markers can be selected from colored marks, textured marks, geometrical patterns, retro-reflective markers, coded targets, scale bars.
- The term "reference zone" is understood to mean a spatial area that enables a user (e.g. the surgeon) to get relevant information on the pose of the surgical tool. A reference zone can be the tip of a surgical instrument.
- The term "pose" is understood to mean the position and the spatial orientation.

- The term "capture images of fiducial markers" is understood to mean that, at a given time, each fiducial marker captured by the stereoscopic camera is either entirely visible or partially visible in the images, excluding the case where a fiducial marker is totally obscured (i.e. not visible at all by the stereoscopic camera).

- The term "partially-obscured" is understood to mean that, at a given time, the fiducial marker is partially visible (but not entirely) in the images captured by the stereoscopic camera. This may be due to:

  (i) a partial occultation caused by an interposition of an object or an individual between the fiducial marker and the stereoscopic camera;
  (ii) a partial occultation caused by a covering of the fiducial marker (e.g. blood spatter, mechanical defect, mechanical damage etc.)

- The term "data processor" is understood to mean a device, such a calculator or a computer, that performs operations on data.

- The term "generate" is understood to mean either a raw (gross) production of patterns (or sub-patterns) that may or may not be subjected to a further selection, or an already filtered production of patterns (or sub-patterns) that has undergone a selection.

- The term "including N fiducial markers" is understood to mean that the set of patterns generated in step b), taken as a whole, covers N fiducial markers where N is an integer inferior or equal to $N_{tot}$.

- The term "reference coordinate system" is understood to mean a geometric reference frame used to describe poses of objects that may be obtained by a 3D geometrical reconstruction.

- The term "detect a presence" is understood to mean that the existence of at least one partially-obscured fiducial marker is revealed, but does not necessarily imply that the at least one partially-obscured fiducial marker can be located.

**Brief description of the drawings**

Other features and advantages will become apparent in the detailed description of various embodiments of the invention, the description being accompanied by examples and references to the appended drawings.

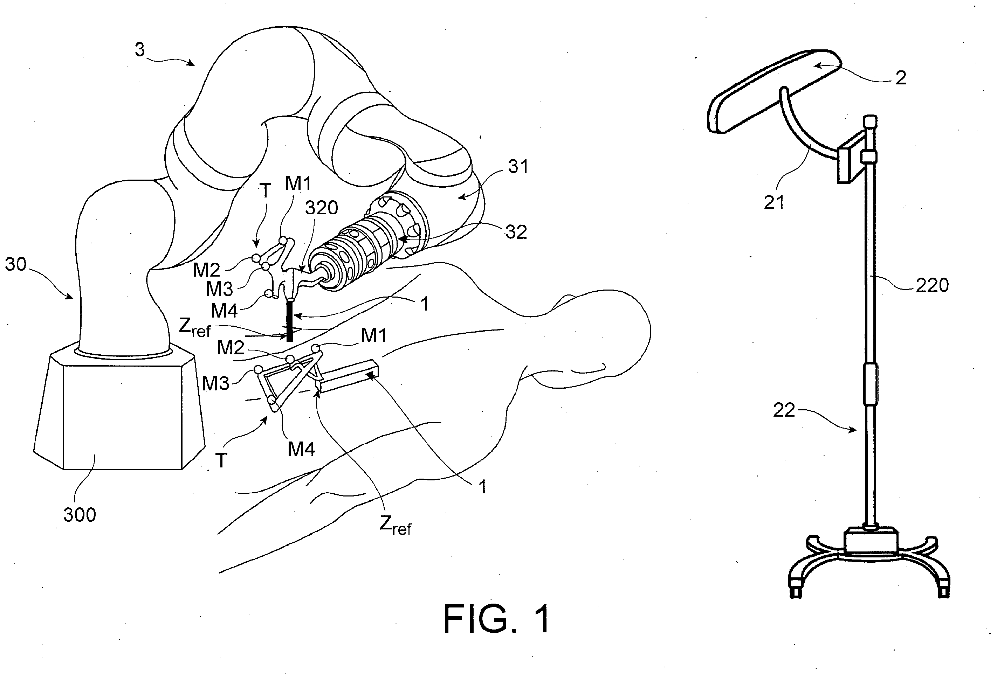

Figure 1 is a partial schematic perspective view of a surgical navigation system according to the invention (the data processor is not illustrated) in a surgical room, illustrating the presence of a robotic arm.

Figure 2 is a flowchart illustrating a first implementation mode of the steps performed by a data processor equipping a surgical navigation system according to the invention.

Figure 3 is a flowchart illustrating a second implementation mode of the steps performed by a data processor equipping a surgical navigation system according to the invention.

Figure 4 is a flowchart illustrating a third implementation mode of the steps performed by a data processor equipping a surgical navigation system according to the invention.

Figure 5 is a flowchart illustrating a fourth implementation mode of the steps performed by a data processor equipping a surgical navigation system according to the invention.

Figure 6 is a flowchart illustrating a fifth implementation mode of the steps performed by a data processor equipping a surgical navigation system according to the invention.

Figure 7 is a flowchart illustrating a sixth implementation mode of the steps performed by a data processor equipping a surgical navigation system according to the invention.

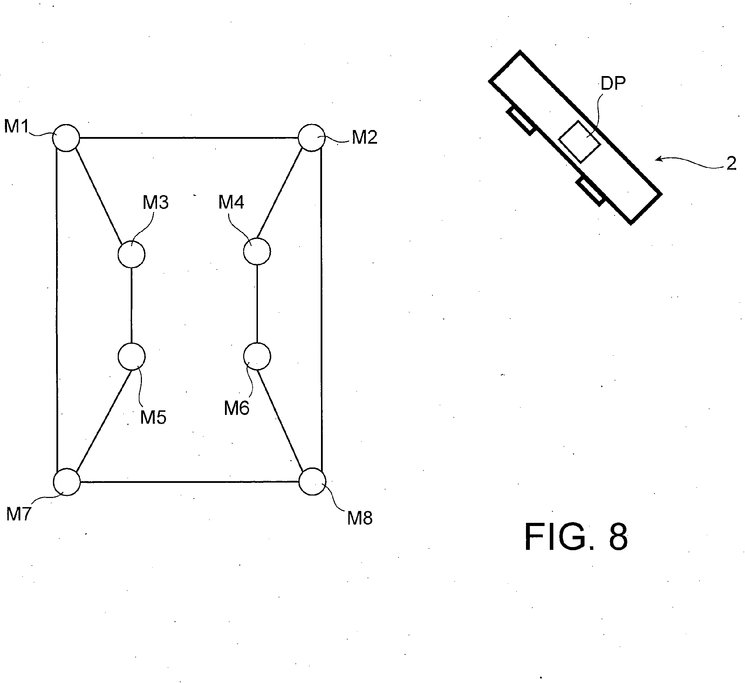

Figure 8 is a schematic top view of $N_{tot}$ (=8) fiducial markers captured by a stereoscopic camera, in the absence of a partially-obscured fiducial marker.

Figure 9 is a schematic top view of a set of 3 patterns (each pattern having 4 fiducial markers) generated by a data processor equipping a surgical navigation according to the invention, illustrating the comparison of the poses of a reference zone computed in step c) therebetween for the set of 3 patterns, in the absence of a partially-obscured fiducial marker.

Figure 10 is a schematic top view of a set of 3 patterns (each pattern having 4 fiducial markers) generated by a data processor equipping a surgical navigation according to the invention, illustrating the comparison of the pose of a reference zone computed in step c') with the poses of the reference zone computed in step c) for the set of 3 patterns, in the absence of a partially-obscured fiducial marker.

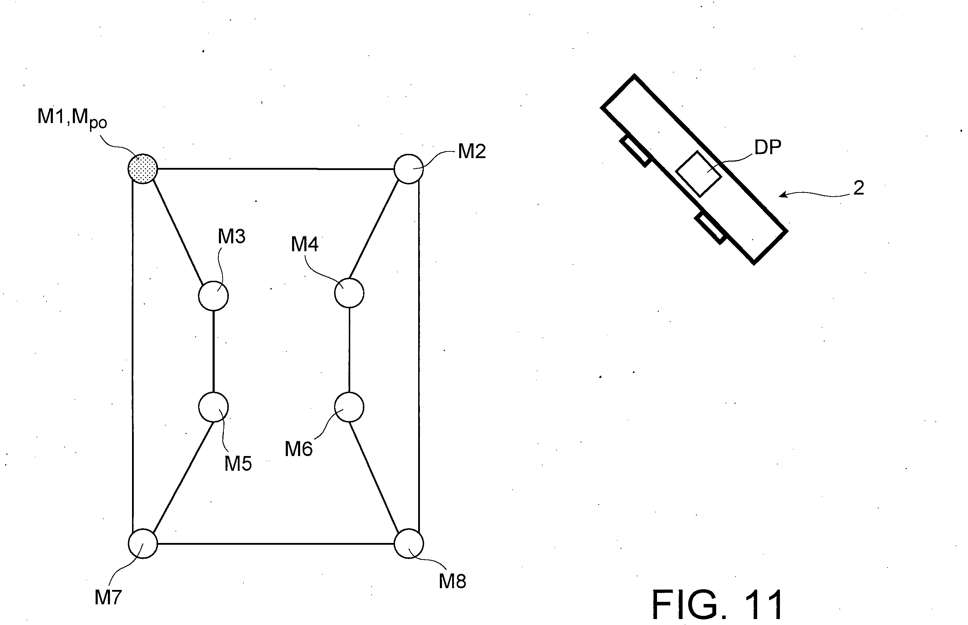

Figure 11 is a schematic top view of $N_{tot}$ (=8) fiducial markers captured by a stereoscopic camera, in the presence of a partially-obscured fiducial marker.

Figure 12 is a schematic top view of a set of 3 patterns (each pattern having 4 fiducial markers) generated by a data processor equipping a surgical navigation according to the invention, illustrating the comparison of the poses of a reference zone computed in step c) therebetween for the set of 3 patterns, in the presence of a partially-obscured fiducial marker.

Figure 13 is a schematic top view of a set of 3 patterns (each pattern having 4 fiducial markers) generated by a data processor equipping a surgical navigation according to the invention, illustrating the comparison of the pose of a reference zone computed in step c') with the poses of the reference zone computed in step c) for the set of 3 patterns, in the presence of a partially-obscured fiducial marker.

Figure 14 is a schematic top view illustrating a generation of a set of 4 sub-patterns (each sub-pattern having 3 fiducial markers) when step e) is performed by a data processor equipping a surgical navigation according to the invention.

Figure 15 is a schematic top view of a set of 4 sub-patterns (each sub-pattern having 3 fiducial markers), illustrating the comparison of the poses of a reference zone computed in step e) therebetween for the set of 4 sub-patterns.

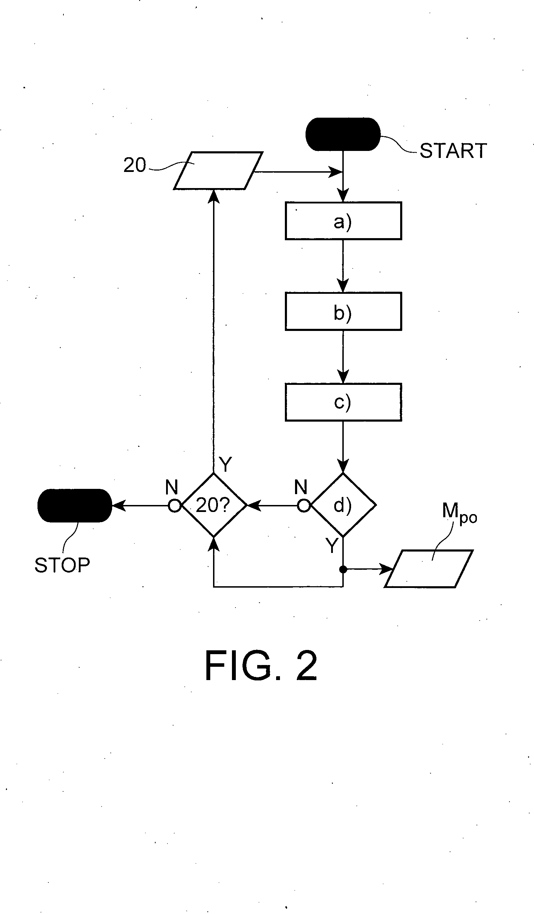

The forms used for Figures 2 to 7 follow the ISO 5807 standard for flowcharts. "Y" means "Yes" i.e. the test result is true. "N" means "No" i.e. the test result is false.

It should be noted that, for the sake of readability and ease of understanding, the drawings described above are schematic and are not necessarily to scale.

**Detailed description of embodiments**

Elements that are identical or provide the same function will carry the same references for the various embodiments, for the sake of simplicity.

One subject of the invention is a surgical navigation system, comprising:

- a surgical tool 1, having a reference zone $Z_{ref}$;
- an optical tracker T, arranged to track a pose of the surgical tool 1, and having fiducial markers M1-M8;
- a stereoscopic camera 2, arranged to capture images 20 of $N_{tot}$ fiducial markers M1-M8, where $N_{tot}$ is an integer superior or equal to 4;
- a data processor DP, configured to perform steps:

  a) identify the $N_{tot}$ fiducial markers M1-M8 and determine the positions of the $N_{tot}$ fiducial markers M1-M8, from the images 20 captured by the stereoscopic camera 2;
  b) generate a set of patterns P1-P3, each pattern P1-P3 having M fiducial markers, where M is an integer superior or equal to 3 and strictly inferior to $N_{tot}$; the set of patterns P1-P3 including N fiducial markers M1-M8, where N is an integer inferior or equal to $N_{tot}$;
  c) for each pattern P1-P3 of the set generated in step b):

    - extract the positions of the corresponding M fiducial markers M1-M8 thereof from step a);
    - compute a pose of the reference zone $Z_{ref}$, in a reference coordinate system, from the extracted positions of the corresponding M fiducial markers M1-M8;

  d) apply a criterion to compare the poses of the reference zone $Z_{ref}$ computed in step c) therebetween for the set of patterns P1-P3, the criterion being designed to detect a presence of at least one partially-obscured fiducial marker $M_{po}$ within the N fiducial markers M1-M8.

Another subject of the invention is a surgical navigation system, comprising:

- a surgical tool 1, having a reference zone $Z_{ref}$;
- an optical tracker T, arranged to track a pose of the surgical tool 1, and having fiducial markers M1-M8;
- a stereoscopic camera 2, arranged to capture images 20 of $N_{tot}$ fiducial markers M1-M8, where $N_{tot}$ is an integer superior or equal to 4;
- a data processor DP, configured to perform steps:

  a) identify the $N_{tot}$ fiducial markers M1-M8 and determine the positions of the $N_{tot}$ fiducial markers M1-M8, from the images 20 captured by the stereoscopic camera 2;
  b) generate a set of patterns P1-P3, each pattern P1-P3 having M fiducial markers M1-M8, where M is an integer superior or equal to 3 and strictly inferior to $N_{tot}$; the set of patterns P1-P3 including N fiducial markers M1-M8, where N is an integer inferior or equal to $N_{tot}$;
  c) for each pattern P1-P3 of the set generated in step b):

    - extract the positions of the corresponding M fiducial markers M1-M8 thereof from step a);
    - compute a pose of the reference zone $Z_{ref}$, in a reference coordinate system, from the extracted positions of the corresponding M fiducial markers M1-M8;

  c') extract the positions of the N fiducial markers M1-M8 from step a); and compute a pose of the reference zone $Z_{ref}$, in the reference coordinate system, from the extracted positions of the N fiducial markers M1-M8;
  d) apply a criterion to compare the pose of the reference zone $Z_{ref}$ computed in step c'), with the poses of the reference zone $Z_{ref}$ computed in step c) for the set of patterns P1-P3, the criterion being designed to detect a presence of at least one partially-obscured fiducial marker $M_{po}$ within the N fiducial markers M1-M8.

### *Surgical tool*

The surgical tool 1 may be an instrument, an implant, or an operating table.

By way of non-limiting example, the surgical tool 1 may be a Cobb spinal elevator. A Cobb spinal elevator is a specialized instrument for use in neurosurgical procedures. It can be used in elevation of spinal components in surgeries that require exploration of the vertebral column. For example, it can be used to detach the ligamentum flavum from the lamina or a vertebral disk from the associated vertebrae. Additionally, a Cobb spinal elevator features a semi-sharp blade as well as a slight curvature making it well suited for an exploration of delicate soft tissue areas with less trauma.

As another example, the surgical tool 1 may be a trocar such as a spine trocar. The surgical tool 1 may be a powered surgical tool such as a powered drill, a powered saw, a powered burr or mill, an ultrasonic milling device, a radiofrequency or microwave or cryogenics ablation needle, or any device that can interact with an anatomical structure to be treated.

As other examples, the surgical tool 1 may be an orthopedic implant such as a pedicular screw, a hip implant, a knee implant, or a shoulder implant.

The surgical tool 1 has a reference zone $Z_{ref}$, which is a spatial area that enables a user (e.g. the surgeon) to get relevant information on the pose of the surgical tool 1. By way of non-limiting example, the reference zone $Z_{ref}$ can be the tip of the surgical tool 1 when the surgical tool 1 is an instrument.

### *Robotic arm*

The surgical tool 1 may be mounted on a robotic arm 3 for assisting a user (e.g. a surgeon) during a surgical intervention. For example, in spine surgery, the user may have to implant one or several screws into at least one vertebra. The robotic arm 3 may assist the user by holding a drill guide and maintaining the drill guide according to a planned axis. The user may thus use a handheld drill passing through the drill guide held by the robotic arm 3 to drill a hole intended to receive the screw in a vertebra along the planned axis. The patient may be equipped with an optical tracker T. The robotic arm 3 may be servo-controlled on the movements of the optical tracker T of the patient in order to maintain alignment with an entry point position on a bone of the patient, compensating for any motion of the bone due to patient's breathing or any mechanical interaction.

The robotic arm 3 may be arranged on a cart. The cart may be mobile on wheels, and may include at least one handle to allow an operator to easily maneuver and transport the robotic arm 3. The cart may be manually actuated or, alternatively, motorized with at least one degree of freedom. At least one of the wheels may be blocked once the cart has been moved to the desired position with respect to the operating table. The cart may comprise switches, such as power switches, an emergency button or the like.

The robotic arm 3 may have:

- a proximal end 30, extending from a base 300;
- a distal end 31, opposite to the proximal end 30.

The robotic arm 3 may be equipped with an end-effector 32 which is attached at the distal end 31. The end-effector 32 may comprise a hand grip and a tool guide 320. The hand grip is configured to be held by a user's hand for manipulating the robotic arm 3. The hand grip may comprise first and second switches configured to be separately actuated by the user to trigger the operating modes of the robotic arm 3. Said switches may include push-buttons, resistive switches, piezoelectric switches, etc. Although said switches may be arranged in another part of the robotic arm 3, said switches are preferably arranged on the hand grip so that the user may use a single hand to actuate one of said switches and at the same time handle the end-effector 32 either to move the robotic arm 3 (in a hand guiding mode) or to follow the movement of the robotic arm 3 (in a computed trajectory mode). The hand grip may comprise at least one user interface configured to provide information about the current mode. Although said user interface may be arranged in another part of the robotic arm 3, said user interface is advantageously located on the hand grip or on the vicinity thereof so as to allow the user to concentrate on the end-effector 32 when the robotic arm 3 is operated. The user interface may include a display with a changing graphical item (e.g. text, marks with variable color, etc.) depending on the current mode. The user interface may include a plurality of light-emitting diodes configured to have predetermined colors and/or blinking conditions depending on the current mode. Said light-emitting diodes may be arranged as a ring around the hand grip or in the vicinity thereof.

The robotic arm 3 may be equipped with a controller, configured to controllably move the robotic arm 3 according to a planned trajectory. The controller may comprise a processor, a data storage device, and a communication device. The controller may be configured to controllably move at least part of the cart (e.g. at least one wheel). The controller may be embedded in the cart. As an alternative, the controller may be provided separate from the cart, and may be configured to communicate wirelessly or by wires with the robotic arm 3.

The robotic arm 3 comprises a plurality of degrees of freedom in translation and/or rotation. Usually, the robotic arm 3 comprises at least five, and preferably six or seven motorized degrees of freedom. To that end, the robotic arm 3 comprises a plurality of articulated segments driven by motors. The robotic arm 3 may be for example the LBR Med™ robot provided by KUKA (Germany). This robotic arm 3 may be controlled in an autonomous mode according to desired targets and trajectories. As an alternative, this robotic arm 3 may be manipulated using a collaborative mode (cobot). As another alternative, this robotic arm 3 may be telemanipulated using a master control device. A combination of these different modes may be used on this robotic arm 3.

The robotic arm 3 may be active in the sense that it holds and moves a powered surgical tool 1 that interacts directly with an anatomical structure. On the contrary, the robotic arm 3 may be passive in the sense that it holds a tool guide 320 in a predefined position with respect to the anatomical structure into which a powered surgical tool 1 is inserted by a surgeon. For example, a powered drill may be mounted on the tool guide 320 in order to actively drill a bone along a predefined path until an end-point of a selected linear trajectory is reached. As another example, a powered burr can be used to remove a volume of bone where a tumor has been detected, the robotic arm 3 being controlled to have the burr tip execute a 3D complex path trajectory corresponding to the bone volume to be removed.

### Optical tracker

The optical tracker T is arranged to track a pose of the surgical tool 1. For this purpose, the optical tracker T has fiducial markers M1-M8. The pose of the surgical tool 1 can be defined by three parameters of positions and three parameters of orientation. By way of non-limiting example, the fiducial markers M1-M8 may be reflective markers designed to reflect infrared radiations. The reflective markers may have a spherical shape.

The optical tracker T may be removably or permanently attached on the surgical tool 1, for example by a mechanical or a magnetic attachment. The optical tracker T may be arranged on the tool guide 320 to track a pose of the surgical tool 1 from a known longitudinal axis which is the guiding axis of the tool guide 320. The optical tracker T may be removably or permanently attached on the tool guide 320, for example by a mechanical or a magnetic attachment.

### Stereoscopic camera

The stereoscopic camera 2 makes it possible to provide depth information in a compact way.

The stereoscopic camera 2 may incorporate a light source (for compactness reasons), configured to emit infrared radiations. The light source may comprise a set of light-emitting diodes. As an alternative, the light source may belong to a dedicated infrared illuminator.

The stereoscopic camera 2 is advantageously configured to detect infrared radiations. The stereoscopic camera is arranged to capture images 20 of $N_{tot}$ fiducial markers M1-M8, where $N_{tot}$ is an integer superior or equal to 4. The stereoscopic camera 2 is advantageously arranged to detect the infrared radiations which are reflected by the reflective markers of the optical tracker T.

The captured images 20 of the $N_{tot}$ fiducial markers M1-M8 may be 2D images in shades of gray or in black and white (by applying a saturation threshold).

The stereoscopic camera 2 may comprise a support 21 mounted on a rolling stand 22. The rolling stand 22 may include a telescopic rod 220 defining an axis. The support 21 of the stereoscopic camera 2 is advantageously mobile in rotation about the axis of the telescopic rod 220. The support 21 of the stereoscopic camera 2 is advantageously mobile in translation along the axis of the telescopic rod 220.

### Data processor

The data processor DP is a device, such a calculator or a computer, that performs operations on data. The data processor DP is advantageously integrated in the stereoscopic camera 2.

### Step a)

The data processor DP is configured to perform step a) identify the $N_{tot}$ fiducial markers M1-M8 and determine the positions of the $N_{tot}$ fiducial markers M1-M8, from the images 20 captured by the stereoscopic camera 2.

By way of non-limiting example, the data processor DP may implement a localization algorithm known from prior art, described notably in the document US 5,828,770.

### Step b)

The data processor DP is configured to perform step b) generate a set of patterns P1-P3, each pattern P1-P3 having M fiducial markers, where M is an integer superior or equal to 3 and strictly inferior to $N_{tot}$; the set of patterns P1-P3 including N fiducial markers M1-M8, where N is an integer inferior or equal to $N_{tot}$. The set of patterns generated in step b), taken as a whole, covers N fiducial markers where N is an integer inferior or equal to $N_{tot}$. In other words, the set of patterns generated in step b), taken as a whole, covers all or part of the $N_{tot}$ fiducial markers M1-M8 captured by the stereoscopic camera 2.

Each pattern P1-P3 of the set generated in step b) is advantageously selected according to the positions of the corresponding M fiducial markers M1-M8 thereof with respect to each other. In other words, the relative positions of the M fiducial markers M1-M8 of a given pattern P1-P3 are compared therebetween in order to select the given pattern P1-P3. For this purpose, the set of patterns P1-P3 generated in step b) advantageously satisfies a spread criterion that compares relative positions of the M fiducial markers M1-M8 for each pattern P1-P3 of the set, from the positions determined in step a); the spread criterion being designed to detect a minimal spatial spread between the M fiducial markers M1-M8 of a given pattern P1-P3 of the set. By way of non-limiting example, the relative positions of the M fiducial markers M1-M8 of a given pattern P1-P3 define a maximum distance between two fiducial markers. The given pattern P1-P3 of the set may be selected if the maximum distance exceeds a first predetermined threshold, corresponding to a first minimal spatial spread. As another example, a secondary distance can be defined between the line formed by said two fiducial markers (defining the maximum distance) and the other fiducial markers of the given pattern P1-P3 of the set. The given pattern P1-P3 may be selected if the secondary distance exceeds a second predetermined threshold, corresponding to a second minimal spatial spread. As an alternative, the given pattern P1-P3 of the set defines a centroid, and may be selected if the distance between its fiducial markers M1-M8 and the centroid exceeds a third predetermined threshold, corresponding to a third minimal spatial spread. As another alternative, the fiducial markers M1-M8 of a given pattern P1-P3 form vertices delimiting a surface. The given pattern P1-P3 may be selected if the surface exceeds an area threshold.

Each pattern P1-P3 of the set generated in step b) is advantageously selected according to the positions of the corresponding M fiducial markers M1-M8 thereof with respect to the stereoscopic camera 2. For this purpose, the set of patterns P1-P3 generated in step b) advantageously satisfies a visibility criterion that compares visibility levels of the M fiducial markers M1-M8 between all patterns P1-P3 of the set, from the images 20 captured by stereoscopic camera 2; the visibility criterion being designed to detect a minimal visibility level at which the M fiducial markers M1-M8 of a given pattern P1-P3 of the set are discerned by the stereoscopic camera 2 within a predetermined margin of error. By way of a non-limiting example, the minimal visibility level may result in a visibility angle, preferably of 50°.

The set of patterns P1-P3 generated in step b) advantageously satisfies both the spread criterion and the visibility criterion.

The data processor DP may be configured to perform step b) by:

$b_1$) setting the integer M of fiducial markers M1-M8 for each pattern P1-P3 of the set;
$b_2$) browsing through all or part of combinations of M fiducial markers M1-M8 from step a), so as to generate patterns P1-P3.

The data processor DP may be configured to perform step:
$b_3$) select a set of patterns P1-P3, from patterns generated in step $b_2$), that satisfies the spread criterion.

As an alternative, the data processor DP may be configured to perform step:
$b'_3$) select a set of patterns P1-P3, from patterns generated in step $b_2$), that satisfies the visibility criterion.

As another alternative, the data processor DP is advantageously configured to perform step:
$b''_3$) select a set of patterns P1-P3, from patterns generated in step $b_2$), that satisfies both the spread criterion and the visibility criterion.

The data processor DP is advantageously configured to perform step b) by setting the integer M of fiducial markers M1-M8 to the integer that is equal to $N_{tot}$-1.

### Step c)

The data processor DP is configured to perform step c) for each pattern P1-P3 of the set generated in step b):

-  extract the positions of the corresponding M fiducial markers M1-M8 thereof from step a);
-  compute a pose of the reference zone $Z_{ref}$, in a reference coordinate system, from the extracted positions of the corresponding M fiducial markers M1-M8.

The pose of the reference zone $Z_{ref}$ may be computed from the extracted positions of the corresponding M fiducial markers M1-M8 by using a matrix transformation between coordinate systems. The matrix transformation may be a rigid transformation referring to geometric transformation of a Euclidean space that preserves the Euclidean distance between every pair of points. The rigid transformation may include rotations, translations, reflections, or a combination thereof.

By way of non-limiting example, the data processor DP may implement Kabsch-Umeyama algorithm in order to compute the pose of the reference zone $Z_{ref}$ from the extracted positions of the corresponding M fiducial markers M1-M8.

### Step c')

The data processor DP may be configured to perform step c') extract the positions of the N fiducial markers M1-M8 from step a); and compute a pose of the reference zone $Z_{ref}$, in the reference coordinate system, from the extracted positions of the N fiducial markers M1-M8.

The pose of the reference zone $Z_{ref}$ may be computed from the extracted positions of the N fiducial markers M1-M8 by using a matrix transformation between coordinate systems. The matrix transformation may be a rigid transformation referring to geometric transformation of a Euclidean space that preserves the Euclidean distance between every pair of points. The rigid transformation may include rotations, translations, reflections, or a combination thereof.

By way of non-limiting example, the data processor DP may implement Kabsch-Umeyama algorithm in order to compute the pose of the reference zone $Z_{ref}$ from the extracted positions of the N fiducial markers M1-M8.

### Step d)

As illustrated in figure 2, in one embodiment, the data processor DP is configured to perform step d) apply a criterion to compare the poses of the reference zone $Z_{ref}$ computed in step c) therebetween for the set of patterns P1-P3, the criterion being designed to detect a presence of at least one partially-obscured fiducial marker $M_{po}$ within the N fiducial markers M1-M8. Step d) is performed by the data processor DP as long as images 20 of $N_{tot}$ fiducial markers M1-M8 are captured by the stereoscopic camera 2. The criterion applied in step d) is advantageously a spread criterion. The spread criterion applied in step d) is advantageously designed to detect a minimal spatial spread between the poses of the reference zone $Z_{ref}$ computed in step c) for the set of patterns P1-P3, so as to detect the presence of the at least one partially-obscured fiducial marker $M_{po}$ within the N fiducial markers M1-M8.

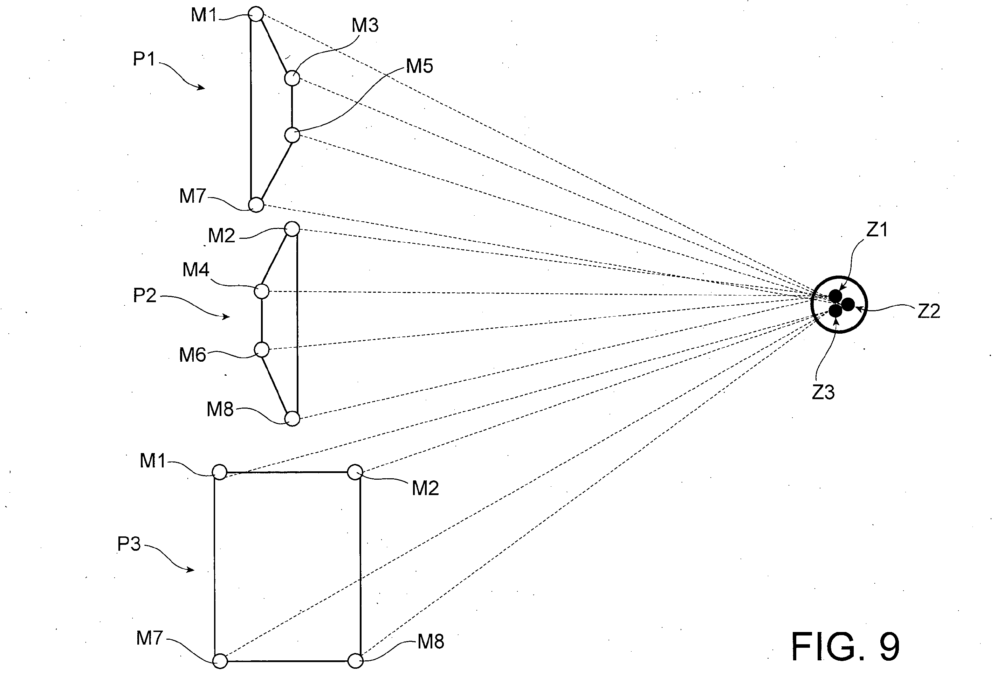

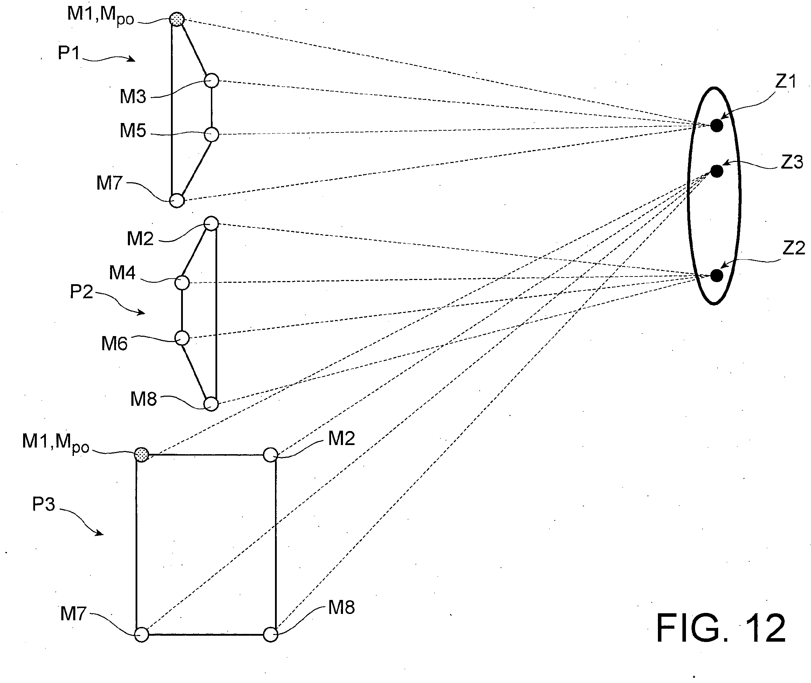

In other words, as illustrated in figures 9 and 12, step d) may be carried out by comparing the positions Z1, Z2, Z3 of the reference zone $Z_{ref}$ computed in step c) therebetween for the set of patterns P1-P3. As illustrated in figure 9, the positions Z1, Z2, Z3 of the reference zone $Z_{ref}$ computed in step c) for the set of patterns P1-P3 do not excess a spread threshold therebetween, indicating the absence of a partially-obscured fiducial marker $M_{po}$. As illustrated in figure 12, the positions Z1, Z2, Z3 of the reference zone $Z_{ref}$ computed in step c) for the set of patterns P1-P3 excess a spread threshold therebetween, indicating the presence of a partially-obscured fiducial marker $M_{po}$.

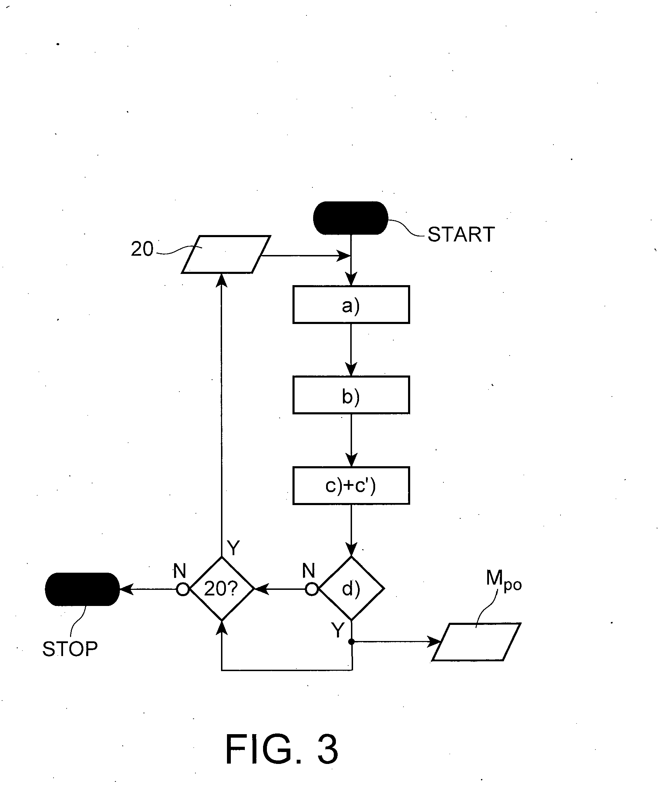

As illustrated in figure 3, in another embodiment, when step c') is performed, the data processor DP is configured to perform step d) apply a criterion to compare the pose of the reference zone $Z_{ref}$ computed in step c'), with the poses of the reference zone $Z_{ref}$ computed in step c) for the set of patterns P1-P3, the criterion being designed to detect a presence of at least one partially-obscured fiducial marker $M_{po}$ within the N fiducial markers M1-M8. Step d) is performed by the data processor DP as long as images 20 of $N_{tot}$ fiducial markers M1-M8 are captured by the stereoscopic camera 2. The criterion applied in step d) is advantageously a spread criterion. When step c') is performed by the data processor DP, the spread criterion applied in step d) is advantageously designed to detect a minimal spatial spread between the pose of the reference zone $Z_{ref}$ computed in step c') and each pose of the reference zone $Z_{ref}$ computed in step c), so as to detect the presence of the at least one partially-obscured fiducial marker $M_{po}$ within the N fiducial markers M1-M8.

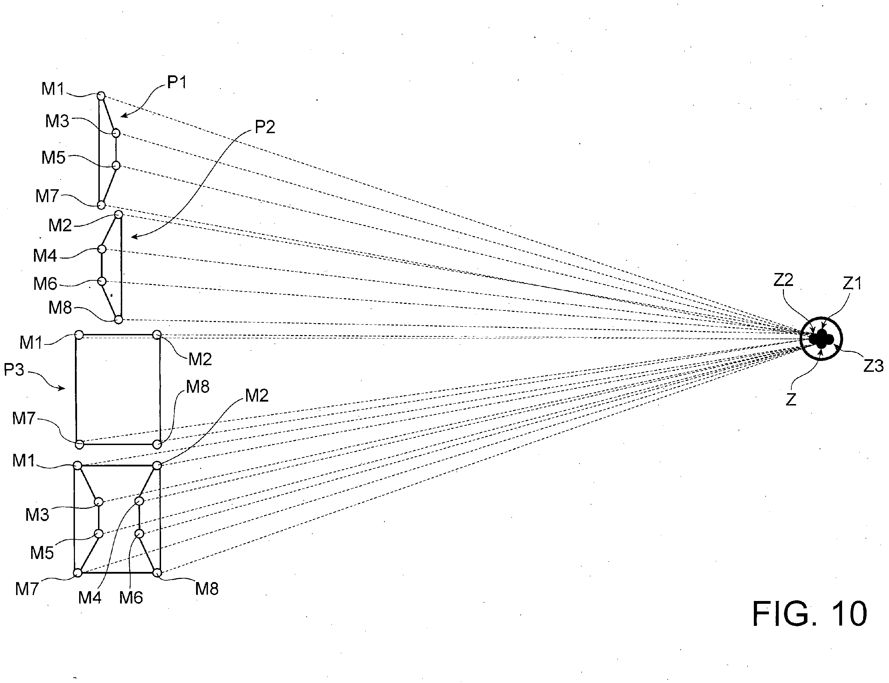

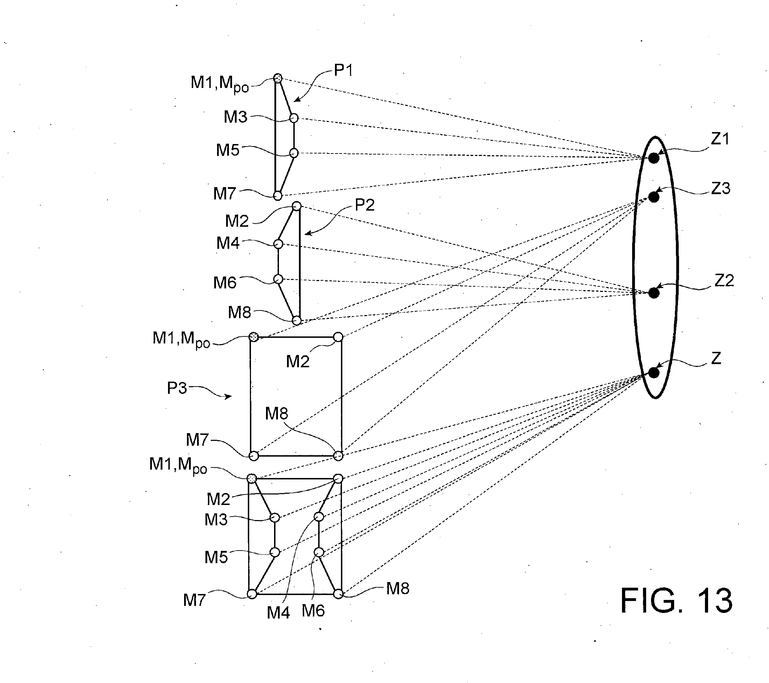

In other words, as illustrated in figures 10 and 13, step d) may be carried out by comparing the position Z of the reference zone $Z_{ref}$ computed in step c'), with the positions Z1, Z2, Z3 of the reference zone $Z_{ref}$ computed in step c) for the set of patterns P1-P3. As illustrated in figure 10, the position Z of the reference zone $Z_{ref}$ computed in step c') and the positions Z1, Z2, Z3 of the reference zone $Z_{ref}$ computed in step c) do not excess a spread threshold, indicating the absence of a partially-obscured fiducial marker $M_{po}$. As illustrated in figure 13, the position Z of the reference zone $Z_{ref}$ computed in step c') and the positions Z1, Z2, Z3 of the reference zone $Z_{ref}$ computed in step c) excess a spread threshold, indicating the presence of a partially-obscured fiducial marker $M_{po}$.

*Step e)*

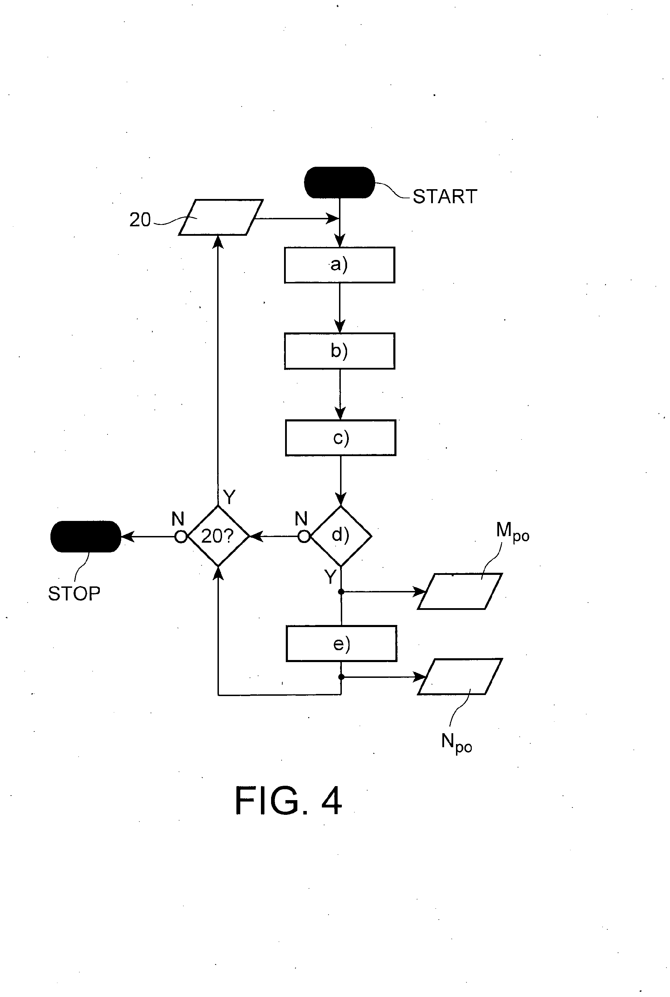

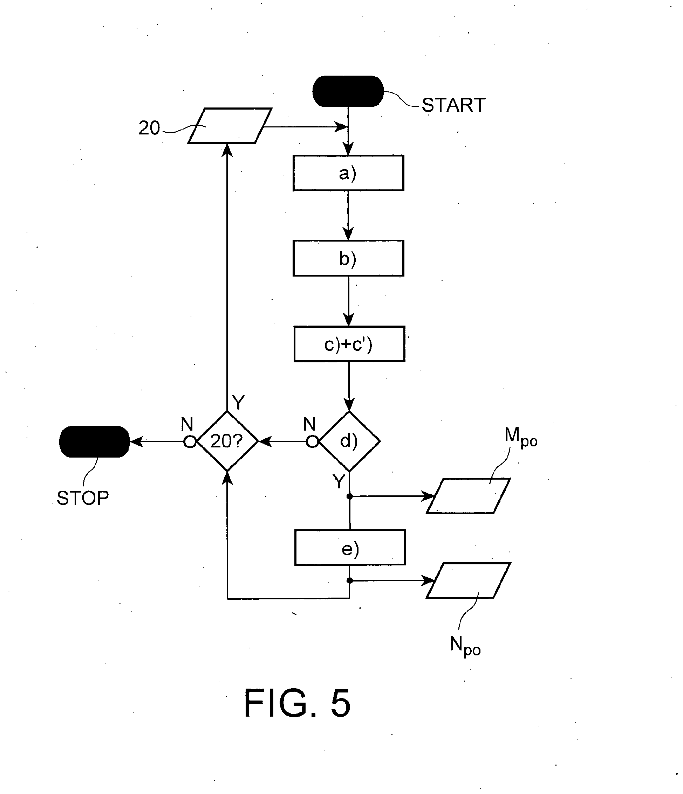

As illustrated in figures 4 and 5, when the following conditions are met:

- the stereoscopic camera 2 is arranged to capture images 20 of $N_{tot}$ fiducial markers M1-M8, where $N_{tot}$ is an integer superior or equal to 5;
- each pattern P1-P3 of the set of patterns generated in step b) has M fiducial markers M1-M8, where M is an integer superior or equal to 4 and strictly inferior to $N_{tot}$;

then the data processor DP is advantageously further configured to perform the following step when the criterion applied in step d) is satisfied:
e) for each pattern P1-P3 of the set generated in step b):

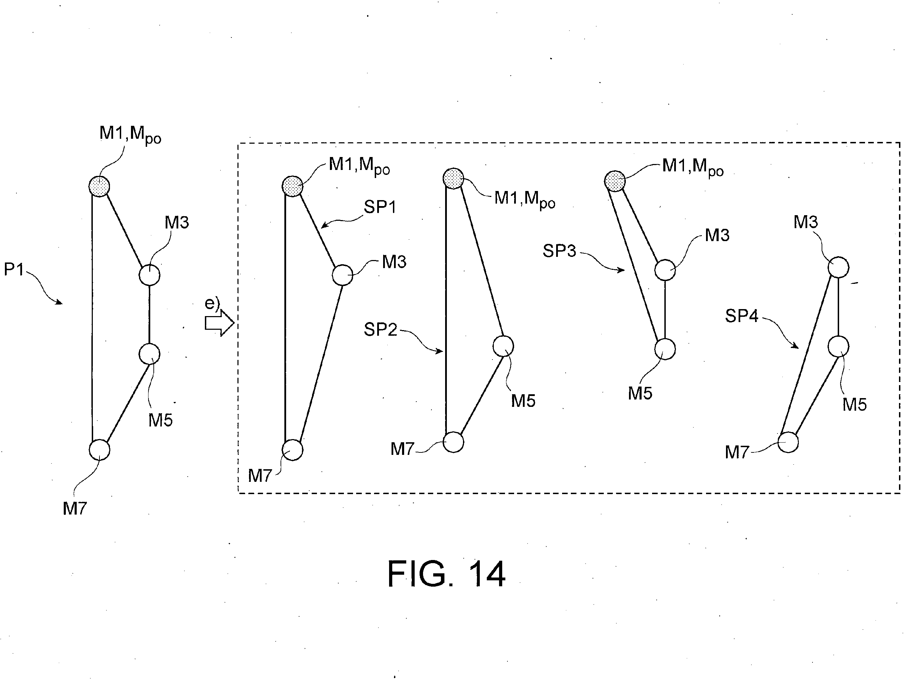

- generate a set of sub-patterns SP1-SP4 (as illustrated in figure 14), each sub-pattern SP1-SP4 having m fiducial markers M1-M8, where m is an integer equal to M-1;
- compute a pose of the reference zone $Z_{ref}$, in the reference coordinate system, for each sub-pattern SP1-SP4 of the set, from the positions of the corresponding m fiducial markers M1-M8 thereof extracted in step c);
- apply a criterion to compare the computed poses of the reference zone $Z_{ref}$ therebetween for the set of sub-patterns SP1-SP4, the criterion being designed to:

   locate $N_{po}$ partially-obscured fiducial markers $M_{po}$ within the M fiducial markers M1-M8 of the corresponding pattern P1-P3, if $N_{po} \leq M-3$, where $N_{po}$ is the number of partially-obscured fiducial markers $M_{po}$;
   detect a presence of $N_{po}$ partially-obscured fiducial markers $M_{po}$ within the M fiducial markers M1-M8 of the corresponding pattern P1-P3, if $N_{po} > M-3$.

The pose of the reference zone $Z_{ref}$ may be computed from the extracted positions of the m fiducial markers M1-M8 by using a matrix transformation between coordinate systems. The matrix transformation may be a rigid transformation referring to geometric transformation of a Euclidean space that preserves the Euclidean distance between every pair of points. The rigid transformation may include rotations, translations, reflections, or a combination thereof.

By way of non-limiting example, the data processor DP may implement Kabsch-Umeyama algorithm in order to compute the pose of the reference zone $Z_{ref}$ from the extracted positions of the m fiducial markers M1-M8.

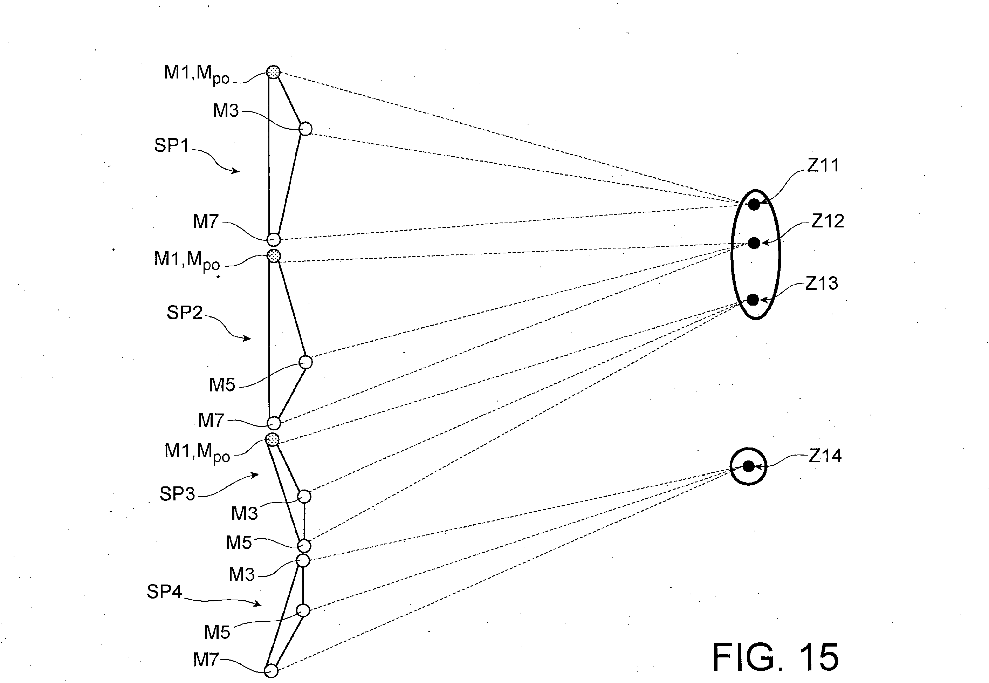

The criterion applied in step e) to compare the computed poses of the reference zone $Z_{ref}$ therebetween for the set of sub-patterns SP1-SP4 is advantageously a spread criterion. As illustrated in figure 15, where $N_{po}$ = M-3, the positions Z11, Z12, Z13, Z14 of the reference zone $Z_{ref}$ computed in step e) for the set of sub-patterns SP1-SP4 excess a spread threshold therebetween. More specifically, the most distant position Z14 is obtained for the sub-pattern SP4 that does not contain the partially-obscured fiducial marker $M_{po}$. Therefore, it is possible to locate the partially-obscured fiducial marker $M_{po}$, which is the fiducial marker M1 of the corresponding pattern P1 that is not present in the sub-pattern SP4.

When the positions Z11, Z12, Z13, Z14 of the reference zone $Z_{ref}$ computed in step e) for the set of sub-patterns SP1-SP4 does not excess a spread threshold therebetween, the existence of $N_{po}$ partially-obscured fiducial markers $M_{po}$, $N_{po}$ > M-3, is revealed, but these partially-obscured fiducial marker cannot be located.

Step e) is performed by the data processor DP as long as images 20 of $N_{tot}$ fiducial markers M1-M8 are captured by the stereoscopic camera 2.

### Steps $f_1$) and $f_2$)

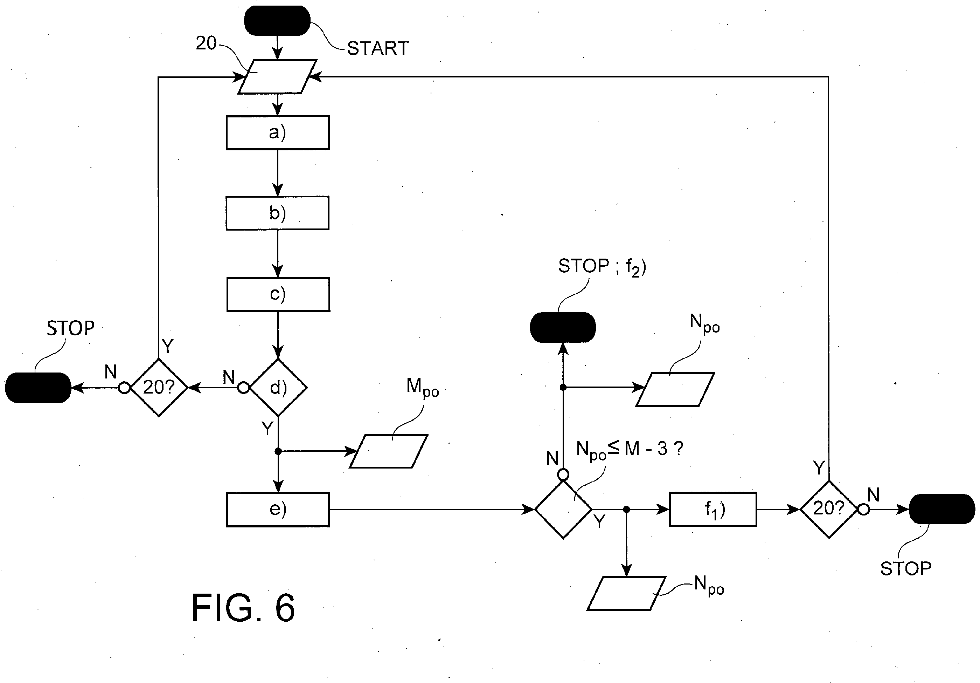

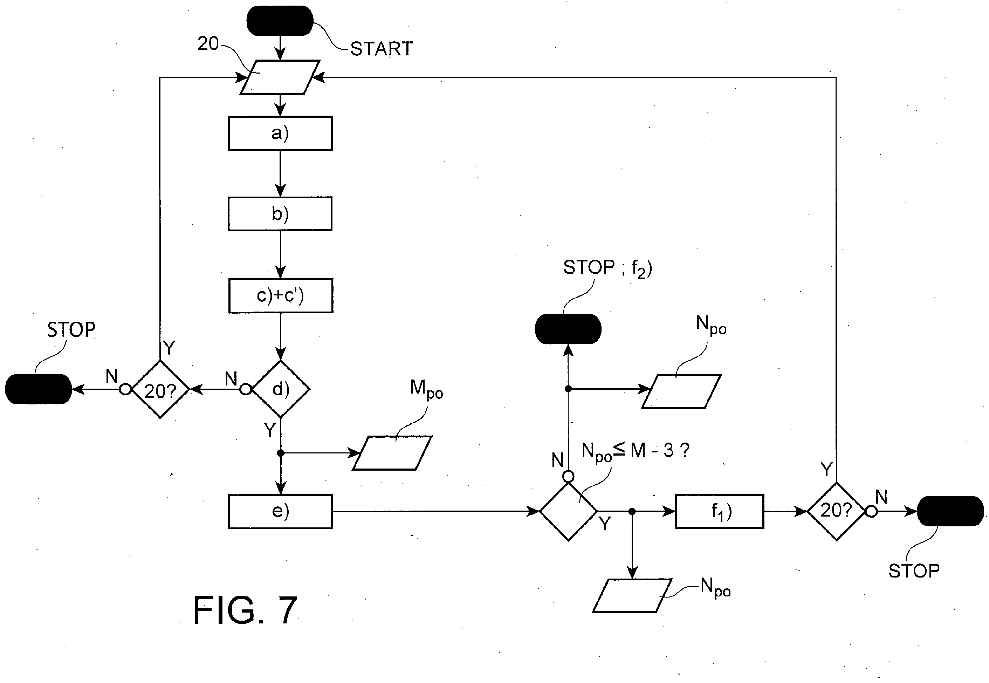

As illustrated in figures 6 and 7, when step e) is performed by the data processor DP, the data processor DP is advantageously further configured to perform steps:

    $f_1$) complete an image registration of the images 20 captured by the stereoscopic camera 2 by removing the $N_{po}$ partially-obscured fiducial markers $M_{po}$ located in step e) when $N_{po} \leq$ M-3;
    $f_2$) stop the surgical navigation if the presence of $N_{po}$ partially-obscured fiducial markers $M_{po}$ is detected in step e) when $N_{po}$ > M-3.

Step $f_1$) is performed by the data processor DP as long as images 20 of $N_{tot}$ fiducial markers M1-M8 are captured by the stereoscopic camera 2.

The invention is not limited to the described embodiments. A person skilled in the art is capable of considering all technically feasible combinations thereof and of substituting them with equivalents.

## Claims

1. A surgical navigation system, comprising:

    - a surgical tool (1), having a reference zone ($Z_{ref}$);
    - an optical tracker (T), arranged to track a pose of the surgical tool (1), and having fiducial markers (M1-M8);
    - a stereoscopic camera (2), arranged to capture images (20) of $N_{tot}$ fiducial markers (M1-M8), where $N_{tot}$ is an integer superior or equal to 4;
    - a data processor (DP), configured to perform step:

        a) identify the $N_{tot}$ fiducial markers (M1-M8) and determine the positions of the $N_{tot}$ fiducial markers (M1-M8), from the images (20) captured by the stereoscopic camera (2);

the surgical navigation system being **characterized in that** the data processor (DP) is configured to perform steps:

        b) generate a set of patterns (P1-P3), each pattern (P1-P3) having M fiducial markers, where M is an integer superior or equal to 3 and strictly inferior to $N_{tot}$; the set of patterns (P1-P3) including N fiducial markers (M1-M8), where N is an integer inferior or equal to $N_{tot}$;
        c) for each pattern (P1-P3) of the set generated in step b):

            - extract the positions of the corresponding M fiducial markers (M1-M8) thereof from step a);
            - compute a pose of the reference zone ($Z_{ref}$), in a reference coordinate system, from the extracted positions of the corresponding M fiducial markers (M1-M8);

        d) apply a criterion to compare the poses of the reference zone ($Z_{ref}$) computed in step c) therebetween for the set of patterns (P1-P3), the criterion being designed to detect a presence of at least one partially-obscured fiducial marker ($M_{po}$) within the N fiducial markers (M1-M8).

2. A surgical navigation system, comprising:

    - a surgical tool (1), having a reference zone ($Z_{ref}$);
    - an optical tracker (T), arranged to track a pose of the surgical tool (1), and having fiducial markers (M1-M8);
    - a stereoscopic camera (2), arranged to capture images (20) of $N_{tot}$ fiducial markers (M1-M8), where $N_{tot}$ is an integer superior or equal to 4;
    - a data processor (DP), configured to perform step:

        a) identify the $N_{tot}$ fiducial markers (M1-M8) and determine the positions of the $N_{tot}$ fiducial markers (M1-M8), from the images (20) captured by the stereoscopic camera (2);

the surgical navigation system being **characterized in that** the data processor (DP) is configured to perform steps:

b) generate a set of patterns (P1-P3), each pattern (P1-P3) having M fiducial markers (M1-M8), where M is an integer superior or equal to 3 and strictly inferior to $N_{tot}$; the set of patterns (P1-P3) including N fiducial markers (M1-M8), where N is an integer inferior or equal to $N_{tot}$;

c) for each pattern (P1-P3) of the set generated in step b):

- extract the positions of the corresponding M fiducial markers (M1-M8) thereof from step a);
- compute a pose of the reference zone ($Z_{ref}$), in a reference coordinate system, from the extracted positions of the corresponding M fiducial markers (M1-M8);

c') extract the positions of the N fiducial markers (M1-M8) from step a); and compute a pose of the reference zone ($Z_{ref}$), in the reference coordinate system, from the extracted positions of the N fiducial markers (M1-M8);

d) apply a criterion to compare the pose of the reference zone ($Z_{ref}$) computed in step c'), with the poses of the reference zone ($Z_{ref}$) computed in step c) for the set of patterns (P1-P3), the criterion being designed to detect a presence of at least one partially-obscured fiducial marker ($M_{po}$) within the N fiducial markers (M1-M8).

3. The surgical navigation system according to claim 1 or 2, wherein:

- the stereoscopic camera (2) is arranged to capture images (20) of $N_{tot}$ fiducial markers (M1-M8), where $N_{tot}$ is an integer superior or equal to 5;
- each pattern (P1-P3) of the set of patterns generated in step b) has M fiducial markers (M1-M8), where M is an integer superior or equal to 4 and strictly inferior to $N_{tot}$;
- the data processor (DP) is further configured to perform the following step when the criterion applied in step d) is satisfied:

e) for each pattern (P1-P3) of the set generated in step b):

- generate a set of sub-patterns (SP1-SP4), each sub-pattern (SP1-SP4) having m fiducial markers (M1-M8), where m is an integer equal to M-1;

- compute a pose of the reference zone ($Z_{ref}$), in the reference coordinate system, for each sub-pattern (SP1-SP4) of the set, from the positions of the corresponding m fiducial markers (M1-M8) thereof extracted in step c);
- apply a criterion to compare the computed poses of the reference zone ($Z_{ref}$) therebetween for the set of sub-patterns (SP1-SP4), the criterion being designed to:

locate $N_{po}$ partially-obscured fiducial markers ($M_{po}$) within the M fiducial markers (M1-M8) of the corresponding pattern (P1-P3), if $N_{po} \leq M-3$, where $N_{po}$ is the number of partially-obscured fiducial markers ($M_{po}$);

detect a presence of $N_{po}$ partially-obscured fiducial markers ($M_{po}$) within the M fiducial markers (M1-M8) of the corresponding pattern (P1-P3), if $N_{po} > M-3$.

4. The surgical navigation system according to claim 3, wherein the data processor (DP) is further configured to perform steps:

$f_1$) complete an image registration of the images (20) captured by the stereoscopic camera (2) by removing the $N_{po}$ partially-obscured fiducial markers ($M_{po}$) located in step e) when $N_{po} \leq M-3$;

$f_2$) stop the surgical navigation if the presence of $N_{po}$ partially-obscured fiducial markers ($M_{po}$) is detected in step e) when $N_{po} > M-3$.

5. The surgical navigation system according to any claim 1 to 4, wherein each pattern (P1-P3) of the set generated in step b) is selected according to the positions of the corresponding M fiducial markers (M1-M8) thereof with respect to each other.

6. The surgical navigation system according to any claim 1 to 5, wherein the set of patterns (P1-P3) generated in step b) satisfies a spread criterion that compares relative positions of the M fiducial markers (M1-M8) for each pattern (P1-P3) of the set, from the positions determined in step a); the spread criterion being designed to detect a minimal spatial spread between the M fiducial markers (M1-M8) of a given pattern (P1-P3) of the set.

7. The surgical navigation system according to any claim 1 to 6, wherein each pattern (P1-P3) of the set generated in step b) is selected according to the positions of the corresponding M fiducial markers (M1-M8) thereof with respect to the stereoscopic camera (2).

8. The surgical navigation system according to any claim 1 to 7, wherein the set of patterns (P1-P3)

generated in step b) satisfies a visibility criterion that compares visibility levels of the M fiducial markers (M1-M8) between all patterns (P1-P3) of the set, from the images (20) captured by stereoscopic camera (2); the visibility criterion being designed to detect a minimal visibility level at which the M fiducial markers (M1-M8) of a given pattern (P1-P3) of the set are discerned by the stereoscopic camera (2) within a predetermined margin of error.

9. The surgical navigation system according to claim 8 in combination with claim 6, wherein the set of patterns (P1-P3) generated in step b) satisfies both the spread criterion and the visibility criterion.

10. The surgical navigation system according to any claim 1 to 9, wherein the data processor (DP) is configured to perform step b) by:

b$_1$) setting the integer M of fiducial markers (M1-M8) for each pattern (P1-P3) of the set;
b$_2$) browsing through all or part of combinations of M fiducial markers (M1-M8) from step a), so as to generate patterns (P1-P3).

11. The surgical navigation system according to claim 10 in combination with claim 6, wherein the data processor (DP) is configured to perform step:
b$_3$) select a set of patterns (P1-P3), from patterns generated in step b$_2$), that satisfies the spread criterion.

12. The surgical navigation system according to claim 10 in combination with claim 8, wherein the data processor (DP) is configured to perform step:
b'$_3$) select a set of patterns (P1-P3), from patterns generated in step b$_2$), that satisfies the visibility criterion.

13. The surgical navigation system according to claim 10 in combination with claim 9, wherein the data processor (DP) is configured to perform step:
b"$_3$) select a set of patterns (P1-P3), from patterns generated in step b$_2$), that satisfies both the spread criterion and the visibility criterion.

14. The surgical navigation system according to any claim 1 to 13, wherein the criterion applied in step d) is a spread criterion.

15. The surgical navigation system according to claim 14, wherein the spread criterion is designed to:

- detect a minimal spatial spread between the poses of the reference zone (Z$_{ref}$) computed in step c) for the set of patterns (P1-P3), when claim 14 depends on claim 1, so as to detect the presence of the at least one partially-ob-

scured fiducial marker (M$_{po}$) within the N fiducial markers (M1-M8);
- detect a minimal spatial spread between the pose of the reference zone (Z$_{ref}$) computed in step c') and each pose of the reference zone (Z$_{ref}$) computed in step c), when claim 14 depends on claim 2, so as to detect the presence of the at least one partially-obscured fiducial marker (M$_{po}$) within the N fiducial markers (M1-M8).

**Patentansprüche**

1. Chirurgisches Navigationssystem, umfassend:

- ein chirurgisches Werkzeug (1), das eine Referenzzone (Z$_{ref}$) aufweist;
- einen optischen Tracker (T), der dazu angeordnet ist, eine Pose des chirurgischen Werkzeugs (1) zu verfolgen, und der Referenzmarker (M1-M8) aufweist;
- eine stereoskopische Kamera (2), die dazu angeordnet ist, Bilder (20) von N$_{tot}$ Referenzmarkern (M1-M8) zu erfassen, wobei N$_{tot}$ eine Ganzzahl größer oder gleich 4 ist;
- einen Datenprozessor (DP), der dazu ausgelegt ist, diesen Schritt durchzuführen:

a) Identifizieren der N$_{tot}$ Referenzmarker (M1-M8) und Bestimmen der Positionen der N$_{tot}$ Referenzmarker (M1-M8) anhand der Bilder (20), die von der stereoskopischen Kamera (2) erfasst werden;

wobei das chirurgische Navigationssystem **dadurch gekennzeichnet ist, dass** der Datenprozessor (DP) dazu ausgelegt ist, diese Schritte durchzuführen:

b) Generieren eines Satzes von Mustern (P1-P3), wobei jedes Muster (P1-P3) M Referenzmarker aufweist, wobei M eine Ganzzahl größer oder gleich 3 ist und zwingend kleiner als N$_{tot}$ ist; wobei der Satz von Mustern (P1-P3) N Referenzmarker (M1-M8) beinhaltet, wobei N eine Ganzzahl kleiner oder gleich N$_{tot}$ ist;
c) für jedes Muster (P1-P3) des in Schritt b) generierten Satzes:

- Extrahieren der Positionen der entsprechenden M Referenzmarker (M1-M8) davon aus Schritt a);
- Berechnen einer Pose der Referenzzone (Z$_{ref}$), in einem Referenzkoordinatensystem, anhand der extrahierten Positionen der entsprechenden M Referenzmarker (M1-M8);

d) Anwenden eines Kriteriums, um die in Schritt c) berechneten Posen der Referenzzone ($Z_{ref}$) für den Satz von Mustern (P1-P3) untereinander zu vergleichen, wobei das Kriterium dazu ausgestaltet ist, das Vorliegen wenigstens eines teilweise verborgenen Referenzmarkers ($M_{po}$) in den N Referenzmarkern (M1-M8) zu detektieren.

2. Chirurgisches Navigationssystem, umfassend:

- ein chirurgisches Werkzeug (1), das eine Referenzzone ($Z_{ref}$) aufweist;
- einen optischen Tracker (T), der dazu angeordnet ist, eine Pose des chirurgischen Werkzeugs (1) zu verfolgen, und der Referenzmarker (M1-M8) aufweist;
- eine stereoskopische Kamera (2), die dazu angeordnet ist, Bilder (20) von $N_{tot}$ Referenzmarkern (M1-M8) zu erfassen, wobei $N_{tot}$ eine Ganzzahl größer oder gleich 4 ist;
- einen Datenprozessor (DP), der dazu ausgelegt ist, diesen Schritt durchzuführen:

a) Identifizieren der $N_{tot}$ Referenzmarker (M1-M8) und Bestimmen der Positionen der $N_{tot}$ Referenzmarker (M1-M8) anhand der Bilder (20), die von der stereoskopischen Kamera (2) erfasst werden;

wobei das chirurgische Navigationssystem **dadurch gekennzeichnet ist, dass** der Datenprozessor (DP) dazu ausgelegt ist, diese Schritte durchzuführen:

b) Generieren eines Satzes von Mustern (P1-P3), wobei jedes Muster (P1-P3) M Referenzmarker (M1-M8) aufweist, wobei M eine Ganzzahl größer oder gleich 3 ist und zwingend kleiner als $N_{tot}$ ist; wobei der Satz von Mustern (P1-P3) N Referenzmarker (M1-M8) beinhaltet, wobei N eine Ganzzahl kleiner oder gleich $N_{tot}$ ist;
c) für jedes Muster (P1-P3) des in Schritt b) generierten Satzes:

- Extrahieren der Positionen der entsprechenden M Referenzmarker (M1-M8) davon aus Schritt a);
- Berechnen einer Pose der Referenzzone ($Z_{ref}$), in einem Referenzkoordinatensystem, anhand der extrahierten Positionen der entsprechenden M Referenzmarker (M1-M8);

c') Extrahieren der Positionen der N Referenzmarker (M1-M8) aus Schritt a); und Berechnen einer Pose der Referenzzone ($Z_{ref}$), in dem Referenzkoordinatensystem, anhand der extrahierten Positionen der N Referenzmarker (M1-M8);

d) Anwenden eines Kriteriums, um die in Schritt c') berechnete Pose der Referenzzone ($Z_{ref}$) mit den Posen der in Schritt c) berechneten Referenzzone $Z_{ref}$ für den Satz von Mustern (P1-P3) zu vergleichen, wobei das Kriterium dazu ausgestaltet ist, das Vorliegen wenigstens eines teilweise verborgenen Referenzmarkers ($M_{po}$) in den N Referenzmarkern (M1-M8) zu detektieren.

3. Chirurgisches Navigationssystem gemäß Anspruch 1 oder 2, wobei:

- die stereoskopische Kamera (2) dazu angeordnet ist, Bilder (20) von $N_{tot}$ Referenzmarkern (M1-M8) zu erfassen, wobei $N_{tot}$ eine Ganzzahl größer oder gleich 5 ist;
- jedes Muster (P1-P3) des Satzes von Mustern, der in Schritt b) generiert wird, weist M Referenzmarker (M1-M8) auf, wobei M eine Ganzzahl größer oder gleich 4 ist und zwingend kleiner als $N_{tot}$ ist;
- der Datenprozessor (DP) ferner dazu ausgelegt ist, den folgenden Schritt durchzuführen, wenn das in Schritt d) angewendete Kriterium erfüllt ist:

e) für jedes Muster (P1-P3) des in Schritt b) generierten Satzes:

- Generieren eines Satzes von Teilmustern (SP1-SP4), wobei jedes Teilmuster (SP1-SP4) m Referenzmarker (M1-M8) aufweist, wobei m eine Ganzzahl gleich M-1 ist;
- Berechnen einer Pose der Referenzzone ($Z_{ref}$), in dem Referenzkoordinatensystem, für jedes Teilmuster (SP1-SP4) des Satzes, anhand der Positionen der entsprechenden m Referenzmarker (M1-M8) davon, die in Schritt c) extrahiert werden;
- Anwenden eines Kriteriums, um die berechneten Posen der Referenzzone ($Z_{ref}$) untereinander für den Satz von Teilmustern (SP1-SP4) zu vergleichen, wobei das Kriterium hierzu ausgestaltet ist:

Lokalisieren von $N_{po}$ teilweise verborgenen Referenzmarkern ($M_{po}$) innerhalb der M Referenzmarker (M1-M8) des entsprechenden Musters (P1-P3), falls $N_{po} \leq$ M-3 ist, wobei $N_{po}$ die Anzahl von teilweise verborgenen Referenzmarkern ($M_{po}$) ist;
Detektieren eines Vorliegens von $N_{po}$ teilweise verborgenen Referenzmarkern ($M_{po}$) innerhalb der M Referenzmarker (M1-M8) des entsprechenden Musters (P1-P3), falls $N_{po} >$ M-3 ist.

**4.** Chirurgisches Navigationssystem gemäß Anspruch 3, wobei der Datenprozessor (DP) ferner dazu ausgelegt ist, diese Schritte durchzuführen:

$f_1$) Abschließen einer Bildregistrierung der Bilder (20), die von der stereoskopischen Kamera (2) erfasst werden, durch Entfernen der $N_{po}$ teilweise verborgenen Referenzmarker ($M_{po}$), die in Schritt e) lokalisiert werden, wenn $N_{po} \leq M-3$ ist;

$f_2$) Anhalten der chirurgischen Navigation, falls das Vorliegen von $N_{po}$ teilweise verborgenen Referenzmarkern ($M_{po}$) in Schritt e) detektiert wird, wenn $N_{po} > M-3$ ist.

**5.** Chirurgisches Navigationssystem gemäß einem der Ansprüche 1 bis 4, wobei jedes Muster (P1-P3) des Satzes, der in Schritt b) generiert wird, gemäß den Positionen der entsprechenden M Referenzmarker (M1-M8) davon in Bezug zueinander ausgewählt wird.

**6.** Chirurgisches Navigationssystem gemäß einem der Ansprüche 1 bis 5, wobei der Satz von Mustern (P1-P3), die in Schritt b) generiert werden, ein Spreizkriterium erfüllt, das relative Positionen der M Referenzmarker (M1-M8) für jedes Muster (P1-P3) des Satzes anhand der in Schritt a) bestimmten Positionen vergleicht; wobei das Spreizkriterium dazu ausgestaltet ist, eine minimale räumliche Spreizung zwischen den M Referenzmarkern (M1-M8) eines gegebenen Musters (P1-P3) des Satzes zu detektieren.

**7.** Chirurgisches Navigationssystem gemäß einem der Ansprüche 1 bis 6, wobei jedes Muster (P1-P3) des Satzes, der in Schritt b) generiert wird, gemäß den Positionen der entsprechenden M Referenzmarker (M1-M8) davon in Bezug auf die stereoskopische Kamera (2) ausgewählt wird.

**8.** Chirurgisches Navigationssystem gemäß einem der Ansprüche 1 bis 7, wobei der Satz von Mustern (P1-P3), die in b) generiert werden, ein Sichtbarkeitskriterium erfüllt, das Sichtbarkeitsstufen der M Referenzmarker (M1-M8) zwischen allen Mustern (P1-P3) des Satzes anhand der Bilder (20), die von der stereoskopischen Kamera (2) erfasst werden, vergleicht; wobei das Sichtbarkeitskriterium dazu ausgestaltet ist, eine minimale Sichtbarkeitsstufe zu detektieren, bei der die M Referenzmarker (M1-M8) eines gegebenen Musters (P1-P3) des Satzes von der stereoskopischen Kamera (2) innerhalb einer vorbestimmten Fehlermarge unterschieden werden.

**9.** Chirurgisches Navigationssystem gemäß Anspruch 8 in Kombination mit Anspruch 6, wobei der in Schritt b) generierte Satz von Mustern (P1-P3) sowohl das Spreizkriterium als auch das Sichtbarkeitskriterium erfüllt.

**10.** Chirurgisches Navigationssystem gemäß einem der Ansprüche 1 bis 9, wobei der Datenprozessor (DP) dazu ausgelegt ist, Schritt b) hierdurch durchzuführen:

$b_1$) Einstellen der Ganzzahl M von Referenzmarkern (M1-M8) für jedes Muster (P1-P3) des Satzes;

$b_2$) Durchsuchen aller oder eines Teils von Kombinationen von M Referenzmarkern (M1-M8) aus Schritt a), um Muster (P1-P3) zu generieren.

**11.** Chirurgisches Navigationssystem gemäß Anspruch 10 in Kombination mit Anspruch 6, wobei der Datenprozessor (DP) dazu ausgelegt ist, diesen Schritt durchzuführen:

$b_3$) Auswählen eines Satzes von Mustern (P1-P3) aus Mustern, die in Schritt $b_2$) generiert werden, der das Spreizkriterium erfüllt.

**12.** Chirurgisches Navigationssystem gemäß Anspruch 10 in Kombination mit Anspruch 8, wobei der Datenprozessor (DP) dazu ausgelegt ist, diesen Schritt durchzuführen:

$b'_3$) Auswählen eines Satzes von Mustern (P1-P3) aus Mustern, die in Schritt $b_2$) generiert werden, der das Sichtbarkeitskriterium erfüllt.

**13.** Chirurgisches Navigationssystem gemäß Anspruch 10 in Kombination mit Anspruch 9, wobei der Datenprozessor (DP) dazu ausgelegt ist, diesen Schritt durchzuführen:

$b''_3$) Auswählen eines Satzes von Mustern (P1-P3) aus Mustern, die in Schritt $b_2$) generiert werden, der sowohl das Spreizkriterium als auch das Sichtbarkeitskriterium erfüllt.

**14.** Chirurgisches Navigationssystem gemäß einem der Ansprüche 1 bis 13, wobei das in Schritt d) angewendete Kriterium ein Spreizkriterium ist.

**15.** Chirurgisches Navigationssystem gemäß Anspruch 14, wobei das Spreizkriterium hierzu ausgestaltet ist:

- Detektieren einer minimalen räumlichen Spreizung zwischen den Posen der Referenzzone ($Z_{ref}$), die in Schritt c) für den Satz von Mustern (P1-P3) berechnet wird, wenn Anspruch 14 von Anspruch 1 abhängt, um das Vorliegen des wenigstens einen teilweise verborgenen Referenzmarkers ($M_{po}$) in den N Referenzmarkern (M1-M8) zu detektieren;

- Detektieren einer minimalen räumlichen Spreizung zwischen der Pose der Referenzzone

($Z_{ref}$), die in Schritt c') berechnet wird, und jeder Pose der Referenzzone ($Z_{ref}$), die in Schritt c) berechnet wird, wenn Anspruch 14 von Anspruch 2 abhängt, um das Vorliegen des wenigstens einen teilweise verborgenen Referenzmarkers ($M_{po}$) in den N Referenzmarkern (M1-M8) zu detektieren.

**Revendications**

1. Système de navigation chirurgicale, comprenant :

   - un outil chirurgical (1), présentant une zone de référence ($Z_{ref}$) ;
   - un suiveur optique (T), agencé pour suivre une pose de l'outil chirurgical (1), et présentant des marqueurs fiduciaires (M1-M8) ;
   - une caméra stéréoscopique (2), agencée pour capturer des images (20) de $N_{tot}$ marqueurs fiduciaires (M1-M8), $N_{tot}$ étant un entier supérieur ou égal à 4 ;
   - un processeur de données (DP), configuré pour effectuer l'étape suivante :

      a) identifier les $N_{tot}$ marqueurs fiduciaires (M1-M8) et déterminer les positions des $N_{tot}$ marqueurs fiduciaires (M1-M8), à partir des images (20) capturées par la caméra stéréoscopique (2) ;

le système de navigation chirurgicale étant **caractérisé en ce que** le processeur de données (DP) est configuré pour effectuer les étapes suivantes :

      b) générer un ensemble de motifs (P1-P3), chaque motif (P1-P3) ayant M marqueurs fiduciaires, M étant un entier supérieur ou égal à 3 et strictement inférieur à $N_{tot}$ ; l'ensemble de motifs (P1-P3) comportant N marqueurs fiduciaires (M1-M8), N étant un entier inférieur ou égal à $N_{tot}$ ;
      c) pour chaque motif (P1-P3) de l'ensemble généré à l'étape b) :

         - extraire les positions des M marqueurs fiduciaires (M1-M8) correspondants de celui-ci provenant de l'étape a) ;
         - calculer une pose de la zone de référence ($Z_{ref}$), dans un système de coordonnées de référence, à partir des positions extraites des M marqueurs fiduciaires (M1-M8) correspondants ;

      d) appliquer un critère pour comparer les poses de la zone de référence ($Z_{ref}$) calculée à l'étape c) entre celles-ci pour l'ensemble des motifs (P1-P3), le critère étant conçu pour détecter la présence d'au moins un marqueur fiduciaire partiellement masqué ($M_{po}$) au sein des N marqueurs fiduciaires (M1-M8).

2. Système de navigation chirurgicale, comprenant :

   - un outil chirurgical (1), présentant une zone de référence ($Z_{ref}$) ;
   - un suiveur optique (T), agencé pour suivre une pose de l'outil chirurgical (1), et présentant des marqueurs fiduciaires (M1-M8) ;
   - une caméra stéréoscopique (2), agencée pour capturer des images (20) de $N_{tot}$ marqueurs fiduciaires (M1-M8), $N_{tot}$ étant un entier supérieur ou égal à 4 ;
   - un processeur de données (DP), configuré pour effectuer l'étape suivante :

      a) identifier les $N_{tot}$ marqueurs fiduciaires (M1-M8) et déterminer les positions des $N_{tot}$ marqueurs fiduciaires (M1-M8), à partir des images (20) capturées par la caméra stéréoscopique (2) ;

le système de navigation chirurgicale étant **caractérisé en ce que** le processeur de données (DP) est configuré pour effectuer les étapes suivantes :

      b) générer un ensemble de motifs (P1-P3), chaque motif (P1-P3) ayant M marqueurs fiduciaires (M1-M8), M étant un entier supérieur ou égal à 3 et strictement inférieur à $N_{tot}$ ; l'ensemble de motifs (P1-P3) comportant N marqueurs fiduciaires (M1-M8), N étant un entier inférieur ou égal à $N_{tot}$ ;
      c) pour chaque motif (P1-P3) de l'ensemble généré à l'étape b) :

         - extraire les positions des M marqueurs fiduciaires (M1-M8) correspondants de celui-ci provenant de l'étape a) ;
         - calculer une pose de la zone de référence ($Z_{ref}$), dans un système de coordonnées de référence, à partir des positions extraites des M marqueurs fiduciaires (M1-M8) correspondants ;

      c') extraire les positions des N marqueurs fiduciaires (M1-M8) provenant de l'étape a) ; et calculer une pose de la zone de référence ($Z_{ref}$), dans le système de coordonnées de référence, à partir des positions extraites des N marqueurs fiduciaires (M1-M8) ;
      d) appliquer un critère pour comparer la pose de la zone de référence ($Z_{ref}$) calculée à l'étape c'), aux poses de la zone de référence ($Z_{ref}$) calculées à l'étape c) pour l'ensemble de motifs (P1-P3), le critère étant conçu pour détecter la présence d'au moins un marqueur fiduciaire partiellement masqué ($M_{po}$) au sein des N marqueurs fiduciaires (M1-M8).

3. Système de navigation chirurgicale selon la revendication 1 ou 2, dans lequel :

   - la caméra stéréoscopique (2) est agencée pour capturer des images (20) de $N_{tot}$ marqueurs fiduciaires (M1-M8), $N_{tot}$ étant un entier supérieur ou égal à 5 ;
   - chaque motif (P1-P3) de l'ensemble de motifs généré à l'étape b) présente M marqueurs fiduciaires (M1-M8), M étant un entier supérieur ou égal à 4 et strictement inférieur à $N_{tot}$ ;
   - le processeur de données (DP) est en outre configuré pour effectuer l'étape suivante lorsque le critère appliqué à l'étape d) est satisfait :

   e) pour chaque motif (P1-P3) de l'ensemble généré à l'étape b) :

   - générer un ensemble de sous-motifs (SP1-SP4), chaque sous-motif (SP1-SP4) ayant m marqueurs fiduciaires (M1-M8), m étant un entier égal à M-1 ;
   - calculer une pose de la zone de référence ($Z_{ref}$), dans le système de coordonnées de référence, pour chaque sous-motif (SP1-SP4) de l'ensemble, à partir des positions des m marqueurs fiduciaires (M1-M8) correspondants de celui-ci extraits à l'étape c) ;
   - appliquer un critère pour comparer les poses calculées de la zone de référence ($Z_{ref}$) entre elles pour l'ensemble des sous-motifs (SP1-SP4), le critère étant conçu pour :

     localiser les $N_{po}$ marqueurs fiduciaires partiellement masqués ($M_{po}$) dans les M marqueurs fiduciaires (M1-M8) du motif (P1-P3) correspondant, si $N_{po} \leq M-3$, $N_{po}$ étant le nombre de marqueurs fiduciaires partiellement masqués ($M_{po}$) ;
     détecter la présence de $N_{po}$ marqueurs fiduciaires partiellement masqués ($M_{po}$) dans les M marqueurs fiduciaires (M1-M8) du motif correspondant (P1-P3), si $N_{po} > M-3$.

4. Système de navigation chirurgicale selon la revendication 3, dans lequel le processeur de données (DP) est en outre configuré pour effectuer les étapes suivantes :

   $f_1$) réaliser un recalage d'images des images (20) capturées par la caméra stéréoscopique (2) en supprimant les $N_{po}$ marqueurs fiduciaires

partiellement masqués ($M_{po}$) situés à l'étape e) lorsque $N_{po} \leq M-3$ ;

   $f_2$) arrêter la navigation chirurgicale si la présence de $N_{po}$ marqueurs fiduciaires partiellement masqués ($M_{po}$) est détectée à l'étape e) lorsque $N_{po} > M-3$.

5. Système de navigation chirurgicale selon l'une quelconque des revendications 1 à 4, dans lequel chaque motif (P1-P3) de l'ensemble généré à l'étape b) est sélectionné selon les positions des M marqueurs fiduciaires (M1-M8) correspondants de celui-ci les uns par rapport aux autres.

6. Système de navigation chirurgicale selon l'une quelconque des revendications 1 à 5, dans lequel l'ensemble de motifs (P1-P3) généré à l'étape b) satisfait un critère d'étalement qui compare des positions relatives des M marqueurs fiduciaires (M1-M8) pour chaque motif (P1-P3) de l'ensemble, à partir des positions déterminées à l'étape a) ; le critère d'étalement étant conçu pour détecter un étalement spatial minimal entre les M marqueurs fiduciaires (M1-M8) d'un motif (P1-P3) donné de l'ensemble.

7. Système de navigation chirurgicale selon l'une quelconque des revendications 1 à 6, dans lequel chaque motif (P1-P3) de l'ensemble généré à l'étape b) est sélectionné selon les positions des M marqueurs fiduciaires (M1-M8) correspondants de celui-ci par rapport à la caméra stéréoscopique (2).

8. Système de navigation chirurgicale selon l'une quelconque des revendications 1 à 7, dans lequel l'ensemble de motifs (P1-P3) généré à l'étape b) satisfait un critère de visibilité qui compare les niveaux de visibilité des M marqueurs fiduciaires (M1-M8) entre tous les motifs (P1-P3) de l'ensemble, à partir des images (20) capturées par une caméra stéréoscopique (2) ; le critère de visibilité étant conçu pour détecter un niveau de visibilité minimal auquel les M marqueurs fiduciaires (M1-M8) d'un motif (P1-P3) donné de l'ensemble sont discernés par la caméra stéréoscopique (2) à l'intérieur d'une marge d'erreur prédéterminée.

9. Système de navigation chirurgicale selon la revendication 8 en combinaison avec la revendication 6, dans lequel l'ensemble de motifs (P1-P3) généré à l'étape b) satisfait à la fois le critère d'étalement et le critère de visibilité.

10. Système de navigation chirurgicale selon une quelconque revendication 1 à 9, dans lequel le processeur de données (DP) est configuré pour effectuer l'étape b) en :

    $b_1$) définissant l'entier M des marqueurs fiduciaires (M1-M8) pour chaque motif (P1-P3) de l'ensemble ;

b$_2$) parcourant tout ou partie des combinaisons de M marqueurs fiduciaires (M1-M8) de l'étape a), de manière à générer des motifs (P1-P3).

11. Système de navigation chirurgicale selon la revendication 10 en combinaison avec la revendication 6, dans lequel le processeur de données (DP) est configuré pour effectuer l'étape suivante :

b$_3$) sélectionner un ensemble de motifs (P1-P3), à partir de motifs générés à l'étape b$_2$), qui satisfont le critère d'étalement.

12. Système de navigation chirurgicale selon la revendication 10 en combinaison avec la revendication 8, dans lequel le processeur de données (DP) est configuré pour effectuer l'étape suivante :

b'$_3$) sélectionner un ensemble de motifs (P1-P3), à partir de motifs générés à l'étape b$_2$), qui satisfait le critère de visibilité.

13. Système de navigation chirurgicale selon la revendication 10 en combinaison avec la revendication 9, dans lequel le processeur de données (DP) est configuré pour effectuer l'étape suivante :

b"$_3$) sélectionner un ensemble de motifs (P1-P3), à partir de motifs générés à l'étape b$_2$), qui satisfait à la fois le critère d'étalement et le critère de visibilité.

14. Système de navigation chirurgicale selon l'une quelconque des revendications 1 à 13, dans lequel le critère appliqué à l'étape d) est un critère d'étalement.

15. Système de navigation chirurgicale selon la revendication 14, dans lequel le critère d'étalement est conçu pour :

- détecter un étalement spatial minimal entre les poses de la zone de référence (Z$_{ref}$) calculée à l'étape c) pour l'ensemble de motifs (P1-P3), lorsque la revendication 14 dépend de la revendication 1, de sorte à détecter la présence de l'au moins un marqueur fiduciaire partiellement masqué (M$_{po}$) au sein des N marqueurs fiduciaires (M1-M8) ;

- détecter un étalement spatial minimal entre la pose de la zone de référence (Z$_{ref}$) calculée à l'étape c') et chaque pose de la zone de référence (Z$_{ref}$) calculée à l'étape c), lorsque la revendication 14 dépend de la revendication 2, de sorte à détecter la présence de l'au moins un marqueur fiduciaire partiellement masqué (M$_{po}$) au sein des N repères fiduciaires (M1-M8).

FIG. 1

FIG. 2

FIG. 3

FIG. 4

FIG. 5

FIG. 6

FIG. 7

FIG. 8

FIG. 9

FIG. 10

EP 4 406 503 B1

FIG. 11

M1,M$_{po}$  M2  M3  M4  M5  M6  M7  M8

DP  2

FIG. 12

EP 4 406 503 B1

FIG. 13

EP 4 406 503 B1

FIG. 14

FIG. 15

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2016242858 A1
- US 2022409288 A1
- US 2021401510 A1
- US 5828770 A